# EUROPEAN PATENT SPECIFICATION

(11) **EP 4 003 327 B1**
(45) Date of publication and mention of the grant of the patent: **25.02.2026**
(21) Application number: 20848440.2
(22) Date of filing: 29.07.2020
(51) Int. Cl.: A61K 31/343, C07D 307/80, C07D 405/10, A61K 9/20, A61K 9/48, A61P 9/00, A61P 13/12, A61P 25/00, A61P 25/28, A61P 37/00, A61P 43/00

(54) **COMPOUNDS FOR USE IN THE TREATMENT OF PAROXYSMAL NOCTURNAL HAEMOGLOBINURIA (PNH)**
VERBINDUNGEN ZUR BEHANDLUNG DER PAROXYSMALEN NÄCHTLICHEN HÄMOGLOBINURIE (PNH)
COMPOSÉS DESTINÉS AU TRAITEMENT DE L'HÉMOGLOBINURIE PAROXYSTIQUE NOCTURNE (HPN)

(30) Priority: 31.07.2019 US 201962881225 P; 25.10.2019 US 201962926175 P; 05.05.2020 US 202063020239 P
(43) Date of publication of application: 01.06.2022
(73) Proprietor: Biocryst Pharmaceuticals, Inc., Durham, North Carolina 27703 (US)
(72) Inventor: BABU, Yarlagadda S., Birmingham, AL 35244 (US); SHERIDAN, William P., Durham, NC 27703 (US)
(74) Representative: EIP
(86) International application number: PCT/US2020/044037
(87) International publication number: WO 2021/021909

(56) References cited:
- WO-A1-2015/009977
- WO-A1-2016/088082
- WO-A1-2019/057946
- WO-A1-2021/072156
- WO-A1-2021/072198
- US-A1- 2013 273 053
- US-A1- 2019 345 135
- US-B1- 6 653 340
- RISITANO ANTONIO M. ET AL: "Anti-complement Treatment for Paroxysmal Nocturnal Hemoglobinuria: Time for Proximal Complement Inhibition? A Position Paper From the SAAWP of the EBMT", FRONTIERS IN IMMUNOLOGY, vol. 10, 14 July 2019 (2019-07-14), pages 1157, XP055791447, DOI: 10.3389/fimmu.2019.01157

## Description

### Related Applications

This application claims the benefit of priority to U.S. Provisional Patent Application No. 62/881,225, filed July 31, 2019; U.S. Provisional Patent Application No. 62/926,175, filed October 25, 2019; and U.S. Provisional Patent Application No. 63/020,239, filed May 5, 2020.

### Background

The complement system is a branch of an organism's immune system that enhances the ability of antibodies and phagocytic cells to destroy and remove foreign particles (e.g., pathogens) from the organism. The complement system comprises a set of plasma proteins that act together to attack extracellular forms of pathogens and induce a series of inflammatory responses to help fight infection. Complement activation can occur through several pathways. For example, complement activation can occur spontaneously in response to certain pathogens or by antibody binding to a pathogen. When complement proteins are activated a cascade is triggered by which one complement protein induces the activation of the next protein in the sequence. The activation of a small number of complement proteins at the start of the pathway is hugely amplified by each successive enzymatic reaction, resulting in the rapid generation of a disproportionately large complement response. (Marrides, S. Pharmacological Reviews 1998, Vol. 50, pages 59-88). In healthy organisms there are regulatory mechanisms to prevent uncontrolled complement activation.

When activated, complement proteins can bind to a pathogen, opsonizing them for engulfment by phagocytes bearing receptors for complement. Then, small fragments of some complement proteins act as chemoattractants to recruit more phagocytes to the site of complement activation, and also to activate these phagocytes. Next, the complement proteins create holes or pores in the invading organisms, leading to their destruction. While complement plays an important role in protecting the body from foreign organisms, it can also destroy healthy cells and tissue. The inappropriate activation of complement is implicated in a long list of disease pathologies (Morgan, B. Eur J Clin Invest 1994, Vol. 24, pages 219-228) affecting the immune, renal, cardiovascular, and neurological systems. Accordingly, there exists a need to develop further complement inhibitors, which have therapeutic potential in the treatment of numerous disorders.

WO2019/057946 discloses multi-cyclic aromatic compounds for use as Factor D modulators.

US2013/273053 discloses compositions for inhibiting MASP-3-dependent complement activation in a subject suffering from paroxysmal nocturnal hemoglobinuria by administering to the subject a composition comprising an amount of a MASP-3 inhibitory agent in an amount effective to inhibit MASP-3-dependent complement activation.

WO2015/009977 discloses compounds for modulating complement alternative pathway activity in a subject for treating a number of diseases and disorders.

WO2016/088082 discloses compounds for treating diseases and disorders mediated by activation of the complement alternative pathway.

US6653340 discloses compounds for use in the complement, coagulation and kallikrein pathways.

### Summary

The invention provides a compound selected from:

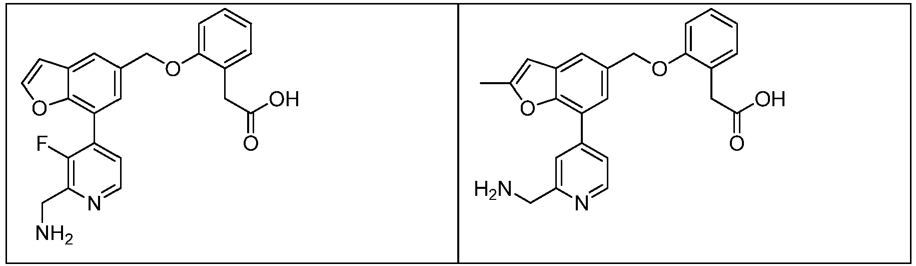

, or a pharmaceutically acceptable salt thereof, for use in a method of treating or preventing paroxysmal nocturnal haemoglobinuria (PNH), the method comprising orally administering to a patient in need thereof a therapeutically effective amount of the compound, or a pharmaceutically acceptable salt thereof;
wherein the PNH is characterized by one or more of hemolytic anemia, thrombosis, and impaired bone marrow function.

### Brief Description of the Drawings

**Figure 1** shows that a compound for use in the invention exhibits dose proportionality in plasma concentration-time curves following oral administration of single escalating doses from 10 mg to 1200 mg in Part 1 in healthy subjects (arithmetic mean ± SD).
**Figure 2** shows that exposure (Cₘₐₓ) to a compound for use in the invention across the single ascending oral dose cohorts of Part 1 was approximately linear and dose-proportional.
**Figure 3** shows that exposure [AUC₍₀₋₂₄₎] to a compound for use in the invention across the single ascending oral dose cohorts of Part 1 was approximately linear and dose-proportional.
**Figure 4** shows dose-dependent inhibition of AP Wieslab activity was detected following oral administration of single ascending dose from 10 mg to 1200 mg of a compound for use in the invention in healthy subjects in Part 1. A >90% suppression of AP activity that was sustained for at least 12 hours post-dose was observed with a single 100 mg or higher oral dose of a compound for use in the invention.
**Figure 5** shows dose-dependent inhibition of AP hemolysis was detected following oral administration of single ascending doses from 10 mg to 1200 mg of a compound for use in the invention in healthy subjects in Part 1. A >90% suppression of AP activity that was sustained for at least 12 hours post-dose was observed with a single 100 mg or higher oral dose of a compound for use in the invention.
**Figure 6** shows steady state PK and PD (AP Wieslab) profiles 12 hours post-dose on Day 3 (Cohorts 4 and 5) or Day 7 (Cohorts 1 and 2) following oral administration of multiple ascending doses from 100 mg/day to 800 mg/day of a compound for use in the invention in healthy subjects in Part 2. Dose-dependent exposure and dose-dependent inhibition of AP Wieslab activity were observed. A > 90% inhibition of AP activity was observed for all multiple ascending doses for at least 12 hours following administration of the final dose.
**Figure 7A** shows that exposure (Cₘₐₓ) to a compound for use in the invention across the multiple ascending oral dose cohorts of Part 2 (Cohorts 1, 2, 4, and 5) was approximately linear and dose-proportional.
**Figure 7B** shows that exposure (AUCₜₐᵤ) to a compound for use in the invention across the multiple ascending oral dose cohorts of Part 2 (Cohorts 1, 2, 4, and 5) was approximately linear and dose-proportional.
**Figure 8A** shows the individual (n=10) PD profiles of a compound for use in the invention (AP Wieslab activity) in healthy subjects through 24 hours post-dose on Day 7 for Cohort 1 of Part 2.
**Figure 8B** shows the individual (n=10) PD profiles of a compound for use in the invention (AP Wieslab activity) in healthy subjects through 24 hours post-dose on Day 7 for Cohort 2 of Part 2.
**Figure 8C** shows the individual (n=10) PD profiles of a compound for use in the invention (AP Wieslab activity) in healthy subjects through 24 hours post-dose on Day 3 for Cohort 4 of Part 2.
**Figure 8D** shows the individual (n=10) PD profiles of a compound for use in the invention (AP Wieslab activity) in healthy subjects through 24 hours post-dose on Day 3 for Cohort 5 of Part 2.
**Figure 8E** shows steady state PD (AP Wieslab) profiles 24 hours post-dose on Day 3 (Cohorts 4 and 5) or Day 7 (Cohorts 1 and 2) following oral administration of multiple ascending doses from 100 mg/day to 800 mg/day of a compound for use in the invention in healthy subjects in Part 2. A > 90% inhibition of AP activity was observed for all multiple ascending doses for at least 12 hours following administration of the final dose.
**Figure 9A** shows the individual (n=10) PD profiles of a compound for use in the invention (AP hemolysis) in healthy subjects through 24 hours post-dose on Day 7 for Cohort 1 of Part 2.
**Figure 9B** shows the individual (n=10) PD profiles of a compound for use in the invention (AP hemolysis) in healthy subjects through 24 hours post-dose on Day 7 for Cohort 2 of Part 2.
**Figure 9C** shows the individual (n=10) PD profiles of a compound for use in the invention (AP hemolysis) in healthy subjects through 24 hours post-dose on Day 3 for Cohort 4 of Part 2.
**Figure 9D** shows the individual (n=10) PD profiles of a compound for use in the invention (AP hemolysis) in healthy subjects through 24 hours post-dose on Day 3 for Cohort 5 of Part 2.
**Figure 9E** shows steady state PD (AP hemolysis) profiles 24 hours post-dose on Day 3 (Cohorts 4 and 5) or Day 7 (Cohorts 1 and 2) following oral administration of multiple ascending doses from 100 mg/day to 800 mg/day of a compound for use in the invention in healthy subjects in Part 2. A > 90% inhibition of AP activity was observed for all multiple ascending doses for at least 12 hours following administration of the final dose.
**Figure 10** shows the PD profile of a compound for use in the invention (AP hemolysis and AP Wieslab assay) in healthy subjects at 24 hour (Day 1) PK troughs and Day 3 (Cohorts 4 and 5) or Day 7 (Cohorts 1 and 2) PK troughs for Cohorts 1, 2, 4, and 5 of Part 2.
**Figure 11A** shows the PD profile of a compound for use in the invention (lactate dehydrogenase; upper limit of normal, ULN) through 28 days of dosing in PNH subjects naive to C5-inhibitor treatment (Cohort 1 of Part 3A).
**Figure 11B** shows the PD profile of a compound for use in the invention (absolute reticulocyte count; 10³/µL) through 28 days of dosing in PNH subjects naive to C5-inhibitor treatment (Cohort 1 of Part 3A).
**Figure 11C** shows the PD profile of a compound for use in the invention (total bilirubin; upper limit of normal, ULN) through 28 days of dosing in PNH subjects naïve to C5-inhibitor treatment (Cohort 1 of Part 3A).
**Figure 11D** shows the PD profile of a compound for use in the invention (hemoglobin levels in g/dL) through 28 days of dosing in PNH subjects naive to C5-inhibitor treatment (Cohort 1 of Part 3A).
**Figure 11E** shows the PD profile of a compound for use in the invention (PNH clone size; percent change) through 28 days of dosing in PNH subjects naive to C5-inhibitor treatment (Cohort 1 of Part 3A).

### Detailed Description

Inhibitors of the complement system are useful in therapeutic methods and compositions suitable for use in treating disorders of the immune, renal, cardiovascular, and neurological systems. The compounds disclosed herein are novel, potent, selective, and orally bioavailable small-molecule inhibitors of human factor D. In preclinical *in vitro* studies, compounds for use in the invention suppressed the AP-mediated hemolysis of erythrocytes from patients with PNH and blocked AP-mediated C3 fragment deposition on PNH erythrocytes at low nanomolar drug concentrations. Oral dosing of compounds for use in the invention achieved >95% suppression of AP mediated hemolysis in preclinical in vivo studies of complement activity in monkeys. Therefore, targeting proximal complement inhibition by compounds for use in the invention has the potential to prevent intravascular and avoid extravascular hemolysis associated with C3 fragment deposition specifically caused by C5 inhibition in patients with PNH.

### Definitions

The articles "a" and "an" are used herein to refer to one or to more than one (i.e., to at least one) of the grammatical object of the article. By way of example, "an element" means one element or more than one element.

Unless otherwise defined, all technical and scientific terms used herein have the same meaning as commonly understood by one of ordinary skill in the art to which this invention belongs. Generally, nomenclature used in connection with, and techniques of, chemistry, cell and tissue culture, molecular biology, cell and cancer biology, neurobiology, neurochemistry, virology, immunology, microbiology, pharmacology, genetics and protein and nucleic acid chemistry, described herein, are those well known and commonly used in the art.

The methods and techniques of the present disclosure are generally performed, unless otherwise indicated, according to conventional methods well known in the art and as described in various general and more specific references that are cited and discussed throughout this specification. See, e.g. "Principles of Neural Science", McGraw-Hill Medical, New York, N.Y. (2000); Motulsky, "Intuitive Biostatistics", Oxford University Press, Inc. (1995); Lodish et al., "Molecular Cell Biology, 4th ed.", W. H. Freeman & Co., New York (2000); Griffiths et al., "Introduction to Genetic Analysis, 7th ed.", W. H. Freeman & Co., N.Y. (1999); and Gilbert et al., "Developmental Biology, 6th ed.", Sinauer Associates, Inc., Sunderland, MA (2000). Suitable methods and materials are described below.

Chemistry terms used herein, unless otherwise defined herein, are used according to conventional usage in the art, as exemplified by "The McGraw-Hill Dictionary of Chemical Terms", Parker S., Ed., McGraw-Hill, San Francisco, C.A. (1985).

The term "pharmaceutically acceptable salt" as used herein includes salts derived from inorganic or organic acids including, for example, hydrochloric, hydrobromic, sulfuric, nitric, perchloric, phosphoric, formic, acetic, lactic, maleic, fumaric, succinic, tartaric, glycolic, salicylic, citric, methanesulfonic, benzenesulfonic, benzoic, malonic, trifluoroacetic, trichloroacetic, naphthalene-2-sulfonic, and other acids. Pharmaceutically acceptable salt forms can include forms wherein the ratio of molecules comprising the salt is not 1: 1. For example, the salt may comprise more than one inorganic or organic acid molecule per molecule of base, such as two hydrochloric acid molecules per molecule of a compound for use in the invention. As another example, the salt may comprise less than one inorganic or organic acid molecule per molecule of base, such as two molecules of a compound for use in the invention per molecule of tartaric acid.

The terms "carrier" and "pharmaceutically acceptable carrier" as used herein refer to a diluent, adjuvant, excipient, or vehicle with which a compound is administered or formulated for administration. Non-limiting examples of such pharmaceutically acceptable carriers include liquids, such as water, saline, and oils; and solids, such as gum acacia, gelatin, starch paste, talc, keratin, colloidal silica, and urea. In addition, auxiliary, stabilizing, thickening, lubricating, flavoring, and coloring agents may be used. Other examples of suitable pharmaceutical carriers are described in Remington's Pharmaceutical Sciences by E.W. Martin.

A "patient," "subject," or "individual" are used interchangeably and refer to either a human or a non-human animal. These terms include mammals, such as humans, primates, livestock animals (including bovines, porcines), companion animals (e.g., canines, felines) and rodents (e.g., mice and rats).

As used herein, a therapeutic that "prevents" or "reduces the risk of developing" a disease, disorder, or condition refers to a compound that, in a statistical sample, reduces the occurrence of the disease, disorder, or condition in the treated sample relative to an untreated control sample, or delays the onset or reduces the severity of one or more symptoms of the disorder or condition relative to the untreated control sample.

In certain embodiments, compounds for use in the invention may be used alone or conjointly administered with another therapeutic agent. The phrases "conjoint administration" and "administered conjointly" refer to any form of administration of two or more different therapeutic compounds such that the second compound is administered while the previously administered therapeutic compound is still effective in the body (*e.g.,* the two compounds are simultaneously effective in the patient, which may include synergistic effects of the two compounds). For example, the different therapeutic compounds can be administered either in the same formulation or in a separate formulation, either concomitantly or sequentially. In certain embodiments, the different therapeutic compounds can be administered within one hour, 12 hours, 24 hours, 36 hours, 48 hours, 72 hours, or a week of one another. Thus, an individual who receives such treatment can benefit from a combined effect of different therapeutic compounds.

In certain embodiments, conjoint administration of compounds for use in the invention with one or more additional therapeutic agent(s) provides improved efficacy relative to each individual administration of the compound for use in the invention or the one or more additional therapeutic agent(s). In certain such embodiments, the conjoint administration provides an additive effect, wherein an additive effect refers to the sum of each of the effects of individual administration of the compound for use in the invention and the one or more additional therapeutic agent(s).

The term "treating" includes prophylactic and/or therapeutic treatments. The term "prophylactic or therapeutic" treatment is art-recognized and includes administration to the subject of one or more of the disclosed compositions. If it is administered prior to clinical manifestation of the unwanted condition (e.g., disease or other unwanted state of the subject) then the treatment is prophylactic (i.e., it protects the subject against developing the unwanted condition), whereas if it is administered after manifestation of the unwanted condition, the treatment is therapeutic (i.e., it is intended to diminish, ameliorate, or stabilize the existing unwanted condition or side effects thereof).

"Administering" or "administration of" a substance, a compound or an agent to a subject can be carried out using one of a variety of methods known to those skilled in the art. For example, a compound or an agent can be administered, intravenously, arterially, intradermally, intramuscularly, intraperitoneally, subcutaneously, ocularly, sublingually, orally (by ingestion), intranasally (by inhalation), intraspinally, intracerebrally, and transdermally (by absorption, e.g., through a skin duct). A compound or agent can also appropriately be introduced by rechargeable or biodegradable polymeric devices or other devices, e.g., patches and pumps, or formulations, which provide for the extended, slow or controlled release of the compound or agent. Administering can also be performed, for example, once, a plurality of times, and/or over one or more extended periods.

Appropriate methods of administering a substance, a compound or an agent to a subject will also depend, for example, on the age and/or the physical condition of the subject and the chemical and biological properties of the compound or agent (e.g., solubility, digestibility, bioavailability, stability and toxicity). In some embodiments, a compound or an agent is administered orally, e.g., to a subject by ingestion. In some embodiments, the orally administered compound or agent is in an extended release or slow release formulation, or administered using a device for such slow or extended release.

An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a composition depends on the composition selected. The compositions can be administered from one or more times per day to one or more times per week; including once every other day. The skilled artisan will appreciate that certain factors may influence the dosage and timing required to effectively treat a subject, including the severity of the disease or disorder, previous treatments, the general health and/or age of the subject, and other diseases present. Moreover, treatment of a subject with a therapeutically effective amount of the compositions described herein can include a single treatment or a series of treatments.

### Oral Dosage Forms of the Disclosure

One aspect of the disclosure provides oral dosage forms of small molecules that inhibit human factor D.

In some embodiments, the oral dosage form comprises a compound selected from:

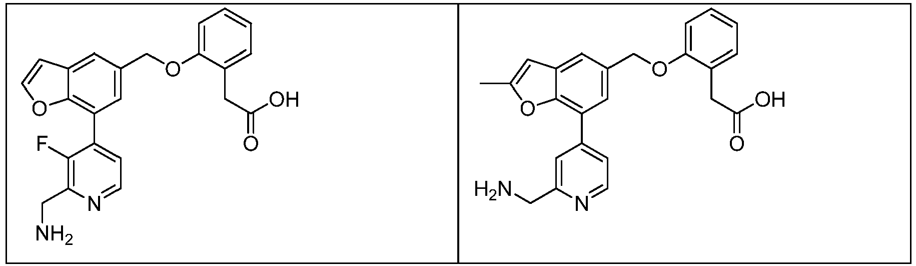

, or a pharmaceutically acceptable salt thereof; and a pharmaceutically acceptable carrier. In certain embodiments, the oral dosage form is a capsule.

Synthetic methods, characterization data, and assay data for the compounds listed above is disclosed in U.S. Provisional Patent Application No. 62/654,108, filed April 6, 2018; International Patent Application No. PCT/US19/26054, filed April 5, 2019; and U.S. Patent Application No. 16/511,642, filed July 15, 2019.

### Pharmaceutical Compositions

The disclosure provides pharmaceutical compositions, each comprising one or more compounds for use in the invention, or pharmaceutically acceptable salts thereof, as described herein, and a pharmaceutically acceptable carrier. In certain embodiments, the pharmaceutical composition comprises a compound for use in the invention, or a pharmaceutically acceptable salt thereof, and a pharmaceutically acceptable carrier. In certain embodiments, the pharmaceutical composition comprises a plurality of compounds for use in the invention, which may include pharmaceutically acceptable salts thereof, and a pharmaceutically acceptable carrier.

In certain embodiments, a pharmaceutical composition for use in the invention further comprises at least one additional pharmaceutically active agent other than a compound for use in the invention. The at least one additional pharmaceutically active agent can be an agent useful in the treatment of a disease or condition characterized by aberrant complement system activity.

Pharmaceutical compositions for use in the invention can be prepared by combining one or more compounds for use in the invention with a pharmaceutically acceptable carrier and, optionally, one or more additional pharmaceutically active agents.

### Methods of Use

The present disclosure provides compounds, and pharmaceutically acceptable salts thereof, that are useful for treating or preventing paroxysmal nocturnal haemoglobinuria (PNH), wherein the PNH is characterized by one or more of hemolytic anemia, thrombosis, and impaired bone marrow function.

In certain aspects, the disclosure provides methods of treating or preventing paroxysmal nocturnal haemoglobinuria (PNH), wherein the PNH is characterized by one or more of hemolytic anemia, thrombosis, and impaired bone marrow function. The method includes the step of orally administering to a subject in need thereof a therapeutically effective amount of a compound for use in the invention, or a pharmaceutically acceptable salt thereof, thereby treating or preventing the PNH. In certain embodiments of treating or preventing PNH characterized by one or more of hemolytic anemia, thrombosis, and impaired bone marrow function, the orally administering suppresses hemolysis of erythrocytes or blocks C3 fragment deposition on erythrocytes or both. In some such embodiments, the suppression is greater than or equal to 50%, such as 80%, 90%, or 95%. In some such embodiments, the PNH is characterized by one or more of the following symptoms: fatigue, erectile dysfunction, headache, and abdominal pain. In certain embodiments of treating or preventing PNH characterized by one or more of hemolytic anemia, thrombosis, and impaired bone marrow function, the orally administering prevents or reduces the occurrence of one or more or all of such symptoms. In some such embodiments, the reduction is greater than or equal to 50%, such as 80%, about 90%, or 95%.

In certain embodiments, the therapeutically effective amount is 1 mg to 6000 mg of the compound or a pharmaceutically acceptable salt thereof, such as 1 mg to 3000 mg, such as 1 mg to 1500 mg, such as 1 mg to 1200 mg, 1 mg to 1000 mg, 1 mg to 800 mg, 1 mg to 600 mg, 1 mg to 400 mg, 1 mg to 300 mg, 1 mg to 200 mg, 1 mg to 100 mg, 10 mg, 30 mg, 100 mg, 200 mg, 300 mg, 400 mg, 600 mg, 800 mg, 1000 mg, or 1200 mg of the compound or a pharmaceutically acceptable salt thereof. In certain embodiments, the therapeutically effective amount is delivered per day.

In certain embodiments, the compound or a pharmaceutically acceptable salt thereof is administered once daily. In other embodiments, the compound or a pharmaceutically acceptable salt thereof is administered twice daily. In still other embodiments, the compound or a pharmaceutically acceptable salt thereof is administered three times daily.

### Formulations, Routes of Administration, and Dosing

The compounds for use in the invention, and pharmaceutically acceptable salts thereof, as described herein, can be formulated as pharmaceutical compositions and administered to a mammalian host, such as a human patient, in a variety of forms adapted to the chosen route of administration, e.g., orally or parenterally, by intravenous, intraperitoneal, intramuscular, topical, or subcutaneous routes. In the invention, the present compounds are administered orally.

Thus, the present compounds may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, and wafers. Such compositions and preparations should contain at least 0.1% of active compound. The percentage of the compositions and preparations may, of course, be varied and may conveniently be between 2% to 60% of the weight of a given unit dosage form. The amount of active compound in such therapeutically useful compositions is such that an effective dosage level will be obtained.

The tablets, troches, pills, and capsules, may also contain the following diluents and carriers: binders such as gum tragacanth, acacia, corn starch or gelatin; excipients such as dicalcium phosphate; a disintegrating agent such as corn starch, potato starch, and alginic acid; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

Useful dosages of the compounds for use in the invention can be determined, at least initially, by comparing their *in vitro* activity and *in vivo* activity in animal models. Methods for the extrapolation of effective dosages in mice, and other animals, to humans are known in the art; for example, see U.S. Pat. No. 4,938,949.

The amount of the compound, or pharmaceutically acceptable salt thereof, required for use in treatment will vary not only with the particular compound, or salt, selected but also with the route of administration, the nature of the condition being treated, and the age and condition of the patient and will be ultimately at the discretion of the attendant physician or clinician.

In general, however, a suitable dose will be in the range of from 0.5 to 100 mg/kg body weight of the recipient per day, e.g., from 3 to 90 mg/kg of body weight per day, from 6 to 75 mg per kilogram of body weight per day, from 10 to 60 mg/kg of body weight per day, or from 15 to 50 mg/kg of body weight per day.

Compounds for use in the invention, or pharmaceutically acceptable salts thereof, can be conveniently formulated in unit dosage form; for example, containing 5 to 1000 mg, 10 to 750 mg, or 50 to 500 mg of active ingredient per unit dosage form. In one embodiment, the disclosure provides a composition comprising a compound for use in the invention, or pharmaceutically acceptable salts thereof, formulated in such a unit dosage form.

In certain aspects, the disclosure provides an oral dosage form comprising the compound, or a pharmaceutically acceptable salt thereof, and a pharmaceutically acceptable carrier. In certain such embodiments, the oral dosage form comprises 1 mg to 1500 mg of the compound or a pharmaceutically acceptable salt thereof, such as 1 mg to 1200 mg, 1 mg to 1000 mg, 1 mg to 800 mg, 1 mg to 600 mg, 1 mg to 400 mg, 1 mg to 300 mg, 1 mg to 200 mg, 1 mg to 100 mg, 10 mg, 30 mg, 100 mg, 200 mg, 300 mg, 400 mg, 600 mg, 800 mg, 1000 mg, or 1200 mg of the compound or a pharmaceutically acceptable salt thereof.

In certain embodiments, the oral dosage form is a capsule. In other embodiments, the oral dosage form is a tablet. In some such embodiments, the tablet is a coated tablet.

The desired dose may conveniently be presented in a single dose or as divided doses to be administered at appropriate intervals, for example, as two, three, four or more sub-doses per day. The sub-dose itself may be further divided, e.g., into a number of discrete loosely spaced administrations. In certain embodiments, the desired dose is administered as a single dose per day. In certain embodiments, the desired dose is administered as divided doses, such as two sub-doses per day or three sub-doses per day.

Compounds for use in the invention, or pharmaceutically acceptable salts thereof, can be administered to patients for treatment for any period of time. Suitable duration of treatment may be based on several factors, such as the disease or condition being treated or other conditions the patient may have, and can be determined by those of ordinary skill in the art. In certain embodiments, the compound for use in the invention, or a pharmaceutically acceptable salt thereof, can be administered for up to 48 weeks, such as 24 weeks, 12 weeks, 6 weeks, 3 weeks, 2 weeks, or 1 week. In certain embodiments, the compound for use in the invention, or a pharmaceutically acceptable salt thereof, can be administered to a patient chronically.

Compounds for use in the invention, or pharmaceutically acceptable salts thereof, can be administered to patients in either the fasted or the fed state (e.g., with or after a high-fat meal).

Compounds for use in the invention, or pharmaceutically acceptable salts thereof, can also be administered in combination with other therapeutic agents, for example, other agents that are useful for treating or preventing a disease or condition characterized by aberrant complement system activity or a disease or condition disclosed herein.

### Example

### INTRODUCTION:

Paroxysmal nocturnal hemoglobinuria (PNH) is a rare, life-threatening disease characterized by hemolytic anemia, thrombosis, and impaired bone marrow function. Uncontrolled activity of the alternative pathway (AP) of complement leads to intravascular hemolysis triggered by the membrane attack complex (C5b-9) formation in patients with PNH. Terminal complement inhibition by IV administration of eculizumab or ravulizumab reduces intravascular hemolysis and is the standard of care for PNH. However, up to one-third of patients treated with eculizumab continue to be transfusion dependent as a result of on-going AP activation and C3-mediated extravascular hemolysis (Kelly RJ, et al. Blood 2011;117(25):6786-6792). The compounds for use in the invention are novel, potent, selective, and orally bioavailable small-molecule inhibitors of human complement factor D. In preclinical in vitro studies, compounds for use in the invention suppressed the AP-mediated hemolysis of erythrocytes from patients with PNH and blocked AP-mediated C3 fragment deposition on PNH erythrocytes at low nanomolar drug concentrations. Oral dosing of the compounds achieved sustained >95% suppression of AP-mediated hemolysis in preclinical in vivo studies of complement activity in monkeys. Therefore, targeting proximal complement inhibition by the compounds for use in the invention will potentially prevent intravascular and avoid extravascular hemolysis associated with opsonization of PNH erythrocytes by C3 in patients with PNH. A first-in-human phase 1 study of compounds for use in the invention in healthy subjects is underway.

### METHODS:

An ongoing randomized, placebo-controlled, phase 1 study is investigating the safety, tolerability, pharmacokinetics (PK), and pharmacodynamics (PD) of single (Part 1) and multiple (Part 2) ascending oral doses of a compound disclosed herein ("Compound") in healthy subjects, and of multiple ascending oral doses of the Compound in subjects with PNH (Part 3). The mean age of all subjects in Parts 1 and 2 was 34.3 years and 50.9% of the subjects were male. Parts 1 and 2 of the study are participant and investigator masked (double blind); Part 3 of the study is not masked.

### Parts 1 and 2:

Healthy male and female subjects were recruited and randomized into cohorts in two Parts.

### Part 1 - Single Ascending Dose Evaluation

Cohort 1, Regimen A: 10 mg Compound or placebo orally, × 1 dose
Cohort 2, Regimen B: 30 mg Compound or placebo orally, × 1 dose
Cohort 3, Regimen C: 100 mg Compound or placebo orally, × 1 dose
Cohort 4, Regimen D, Period 1: 300 mg Compound or placebo orally, × 1 dose (fasted)
Cohort 4, Regimen D, Period 2: 300 mg Compound or placebo orally, × 1 dose (administration after a high fat meal); dosing in Period 2 was separated from dosing in Period 1 by 7 days
Cohort 5, Regimen E: 600 mg Compound or placebo orally, × 1 dose
Cohort 6, Regimen F: 1200 mg Compound or placebo, × 1 dose

Subjects in Part 1 were treated with a single oral dose of study drug (Compound or placebo) per dose cohort. Escalation to the next higher dose level in Part 1 occurred only after satisfactory review of clinical safety and pharmacokinetic data. Subjects in Part 1 Cohort 4 initially received a single dose under fasting conditions; after an adequate washout period, all subjects in Part 1 Cohort 4 were given a second dose after a high-fat meal. Subjects received the same study drug and dose (Compound or placebo) in both dosing periods.

### Part 2 - Multiple Ascending Dose Evaluation

Cohort 1, Regimen G: 100 mg Compound or placebo/day orally, 7 days (13 doses)
Cohort 2, Regimen H: 200 mg Compound or placebo/day orally, 7 days (13 doses)
Cohort 3, Regimen I: 100 mg Compound or placebo/day orally, 14 days (27 doses)
Cohort 4, Regimen J: 400 mg Compound or placebo/day orally, 3 days (5 doses)
Cohort 5, Regimen K: 800 mg Compound or placebo/day orally, 3 days (5 doses)

Subjects in Part 2 were treated with either a 3-, 7- or 14-day course of study drug (Compound or placebo) administered orally. For all cohorts, the daily dose was split into 2 equal doses, administered 12 hours apart (i.e., BID; Q12hr). Subjects received a final morning dose of study drug (Compound or placebo) on the last day of dosing. Escalation to the next higher dose level in Part 2 occurred only after satisfactory review of clinical safety and pharmacokinetic data. Subjects in Part 2 were administered a prophylactic antibiotic or vaccination against Neisseria meningitides (Cohort 3) when Compound was administered.

Safety and tolerability in Parts 1 and 2 were evaluated via clinical and laboratory monitoring. Plasma concentrations of the Compound in Parts 1 and 2 were measured in serial post-dose samples using a validated LC/MS/MS assay. PD effects of the Compound in Parts 1 and 2 was assessed using multiple assays, such as inhibition of serum complement AP activity measured by AP specific Wieslab assay, lysis of rabbit red blood cells by human serum (AP hemolysis assay), and the change in plasma AP pathway biomarker Bb (the split product of complement factor B by the enzymatic action of factor D).

The primary endpoints of Parts 1 and 2 were safety and tolerability of the Compound based on laboratory and examination findings. Secondary endpoints included pharmacokinetic parameters [such as the maximum (peak) plasma drug concentration observed (Cₘₐₓ), median time to reach maximum (peak) plasma drug concentration following drug administration (Tₘₐₓ), the area under the plasma concentration-time curve from time 0 through 24 hours (AUC₀₋₂₄), AUC extrapolated to infinite time (AUC_{inf}), terminal elimination half-life (t_{1/2}), and dose proportionality] and the pharmacodynamic parameters in the paragraph above.

### RESULTS:

### Part 1 - Single Ascending Dose Evaluation

Forty-eight healthy subjects completed dosing in the 6 single ascending dose cohorts (10 mg, 30 mg, 100 mg, 300 mg, 600 mg, and 1200 mg) in Part 1. For all Cohorts of Part 1, 8 subjects were enrolled per Cohort (6 Compound and 2 placebo treated subjects per cohort). Subjects in Cohorts 1-3 were orally administered Compound in a single dose administered as a single capsule containing 10 mg, 30 mg, or 100 mg of Compound, respectively. Subjects in Cohort 4 were orally administered 300 mg Compound in a single dose administered as two 150 mg capsules. Subjects in Cohort 5 were orally administered 600 mg Compound in a single dose administered as three 200 mg capsules. Subjects in Cohort 6 were orally administered 1200 mg Compound in a single dose administered as six 200 mg capsules. Placebo for each Cohort was provided in matching form.

For Cohort 4, Period 1, subjects initially received a first single dose of study drug (Compound or placebo) as described above under fasting conditions (fasted). For Cohort 4, Period 2, after a washout period of 7 days all subjects were given a second single dose of study drug (Compound or placebo) as described above after a high-fat meal (fed). Subjects received the same study drug and dose (Compound or placebo) in both Periods 1 and 2.

Table 1 provides a summary of key pharmacokinetic parameters of Compound in healthy subjects following oral administration of escalating doses of Compound as described above for Part 1.

**Table 1: Geometric mean (CV%) key pharmacokinetic parameters of Compound in healthy subjects following oral administration of escalating doses of Compound in capsules.**

| **Regimen^{a}** | **Tₘₐₓ^{b} (h)** | **Cₘₐₓ (ng/mL)** | **AUC₍₀₋₂₄₎ (ng*h/mL)** | **AUC_{(0-inf)} (ng*h/mL)** | **t_{1/2}^{c} (h)** |
|---|---|---|---|---|---|
| **Cohort 1 (10 mg Compound)** | 1 (0.5-2) | 79.8 (42.2%) | 336 (14.6%) | 357 (53.7%) | 6.47 (23.7%) |
| **Cohort 2 (30 mg Compound)** | 2 (1-2) | 232 (43.4%) | 968 (41.5%) | 999 (41.3%) | 4.51 (10.2%) |
| **Cohort 3 (100 mg Compound)** | 1 (0.5-2) | 869 (42.9%) | 3700 (30.4%) | 3990 (29.3%) | 10.5^{d} (19.4%) |
| **Cohort 4, Period 1 (300 mg Compound, fasted)** | 1.5 (0.5-3) | 1990 (49.7%) | 9540 (31.5%) | 9780 (31.2%) | 4.3 (15.4%) |
| **Cohort 4, Period 2 (300 mg Compound, fed)** | 3.5 (2-5) | 2360 (26.8%) | 12700 (22.2%) | 13000 (22.2%) | 4.2 (12.8%) |
| **Cohort 5 (600 mg Compound)** | 1.5 (0.5-5) | 5600 (22.1%) | 23400 (15.4%) | 24300 (15.3%) | 6.8 (24.3%) |
| **Cohort 6 (1200 mg Compound)** | 2.5 (2-5) | 10350 (43.9%) | 50150 (35.6%) | 52010 (35.3%) | 5.7 (31.2%) |

| | | | | | |
|---|---|---|---|---|---|
| a. All doses were administered in the fasted state unless otherwise specified. b. Median and range. c. Calculated based on PK samples from 0-24 h. d. Calculated based on PK samples from 0-72 hours for most of Cohort 3 and 24 hours for straggler subjects. | | | | | |

Compound administered as ascending oral doses of 10 mg to 1200 mg was safe and generally well tolerated in all subjects in all Cohorts. There were no study discontinuations, no serious adverse events, and no clinically significant changes is safety laboratory parameters. Headache was the most frequently reported treatment-emergent adverse event; incidence of headache had no dose response relationship. All headaches were mild in severity and resolved spontaneously.

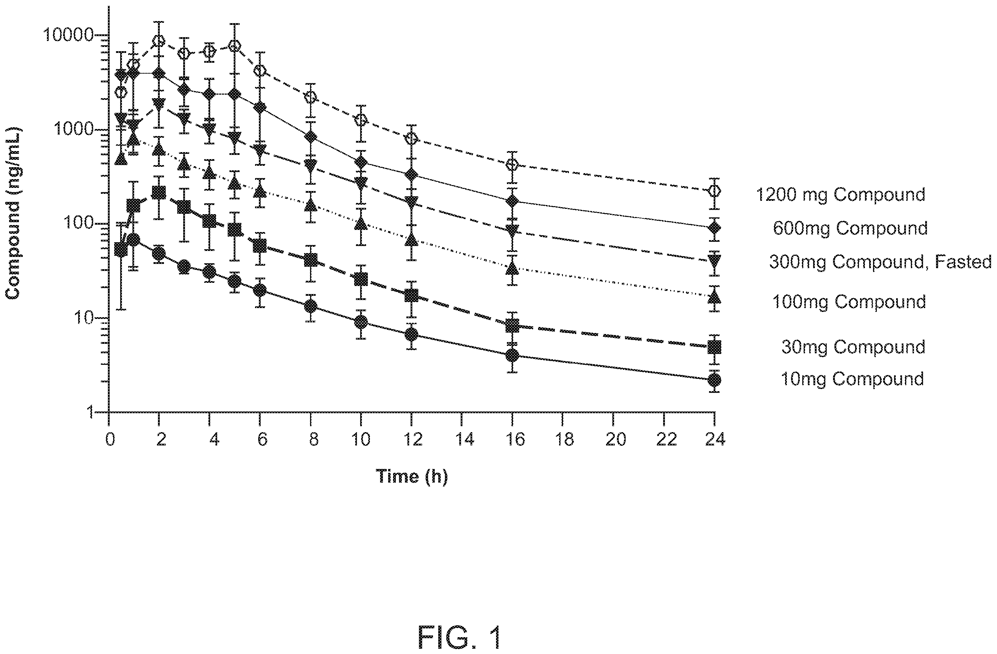

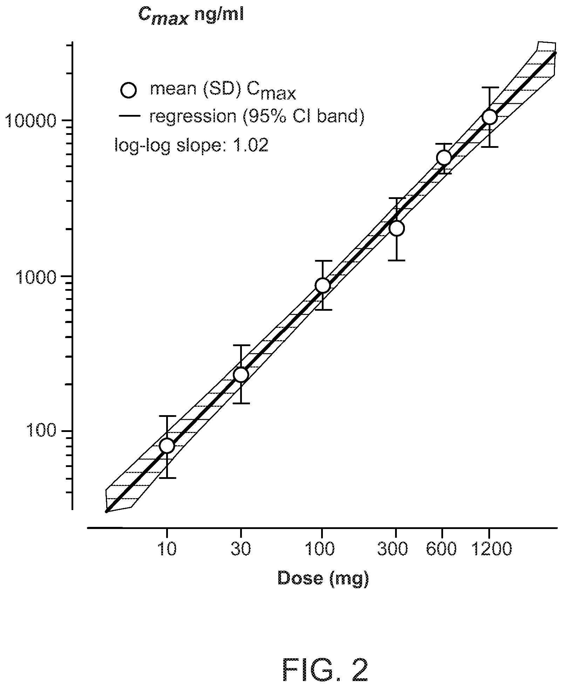

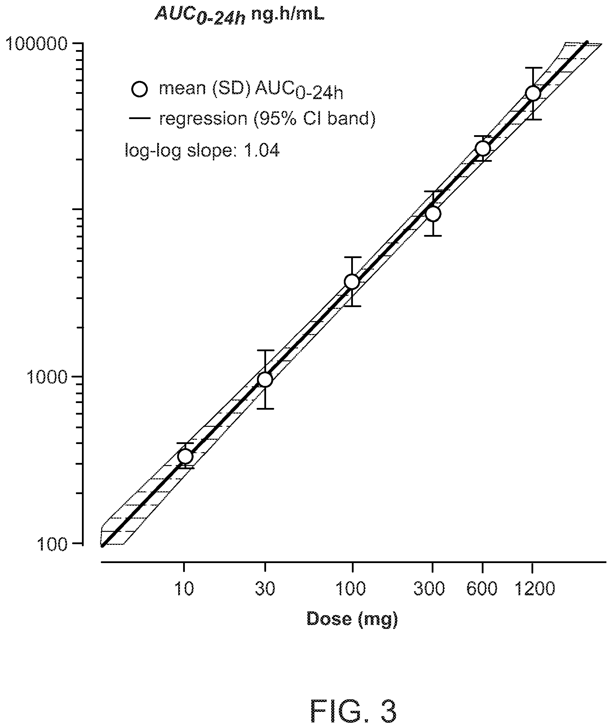

PK parameters of ascending oral doses of Compound for all Cohorts in Part 1 were well characterized over the 24-hour sampling period (Figure 1; Table 1) and approximately linear and dose proportional (Figures 2 and 3). Mean Compound concentration at 12 hours post dose was within or above the anticipated threshold of 8 to 10 times the factor D EC₅₀ for single ascending doses ≥ 300 mg.

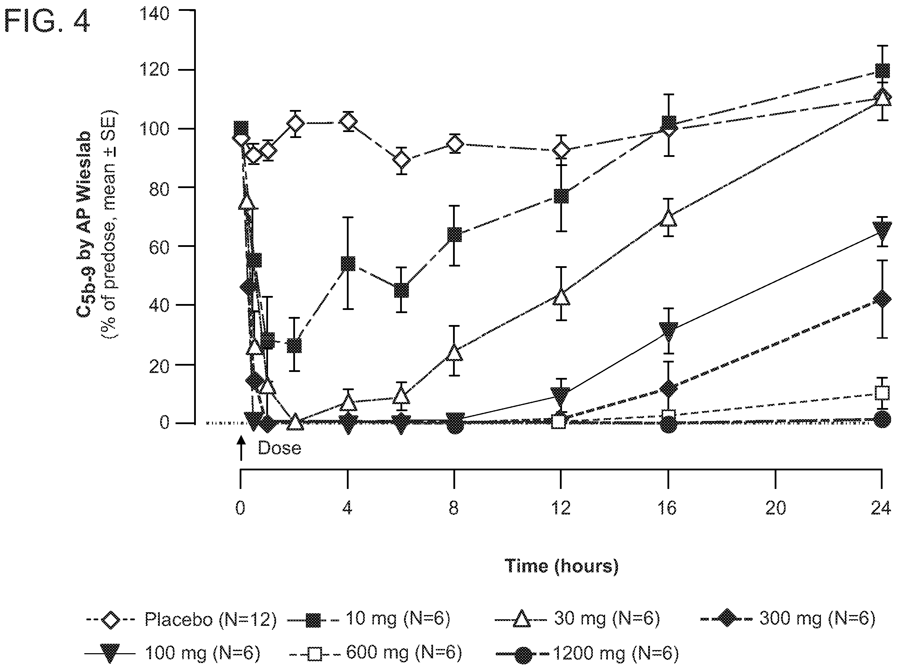

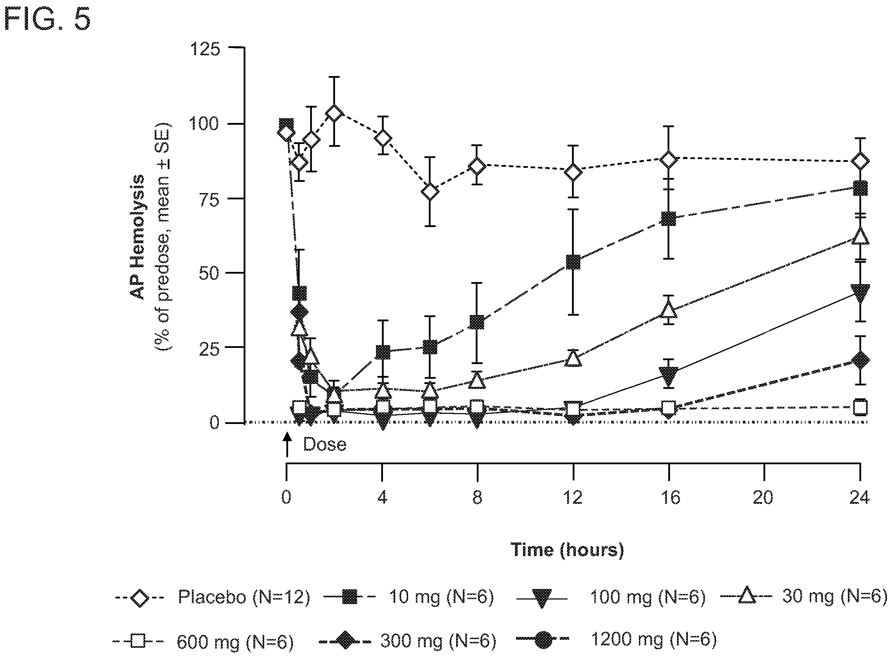

Dose-dependent suppression of the AP complement functional activity was observed in Part 1. Dose-dependent inhibition of AP Wieslab activity was detected, with greater than 90% suppression of AP Wieslab activity sustained for 12 hours post-dose in Cohort 3 (90.4% ± 13.8% inhibition) and Cohort 4 (97.8% ± 4.0% inhibition; data from Period 1) (Figure 4). Approximately 90% suppression of AP Wieslab activity was sustained for over 24 hours post-dose in Cohort 5 (89.7% ± 12.9% inhibition) and Cohort 6 (98.5% ± 2.7% inhibition), with near complete suppression observed for 24 hours post-dose in Cohort 6 (Figure 4). Dose-dependent inhibition of AP hemolysis was also detected with greater than 90% suppression of AP hemolysis sustained for 12 hours post-dose in Cohort 3 (95.0% ± 3.2% inhibition) and Cohort 4 (97.3% ± 2.5% inhibition; data from Period 1) (Figure 5). Approximately 95% suppression of AP hemolysis was sustained for over 24 hours post-dose in Cohort 5 (94.5% ± 4.2% inhibition) and Cohort 6 (95.2% ± 5.3% inhibition) (Figure 5). Figures 4 and 5 shows ≥ 95% suppression of AP activity was observed for single ascending doses ≥ 300 mg for 12 hours following administration, with the duration of suppression increasing with dose. Oral administration of Compound suppressed Factor Bb formation by over 50% in all Cohorts (data not shown). The inhibition values shown above are compared to pre-dose values and are mean values (± SD of the mean) derived from all subjects in each Cohort.

In comparing the exposure of Compound administered after food to that of fasted administration, the median Tₘₐₓ was slightly delayed, and Cₘₐₓ and AUC were slightly higher, yielding a modest increase in exposure following administration with a high-fat meal (Table 1). In light of the minimal impact observed on PK when comparing the fed versus fasted data, dosing for the fifth and sixth cohorts in Part 1 was continued in the fasting state, and Parts 2 amd 3 proceeded in the fasted state.

In summary, for Part 1 Compound administered as a single oral dose of 10 mg to 1200 mg was safe and generally well tolerated in all subjects with no Compound-related safety concerns of note. Furthermore, Compound administered as a single oral dose of 100 mg to 1200 mg demonstrated dose-dependent suppression of the AP functional activity.

### Part 2 - Multiple Ascending Dose Evaluation

Part 2 is currently ongoing. Additional Cohorts in Part 2 may be enrolled to test higher supratherapeutic dosing and/or 1 time per day (i.e., QD) administration in healthy subjects. 55 healthy subjects completed dosing in the 5 multiple ascending dose cohorts (100 mg and 200 mg per day for 7 days, 400 mg and 800 mg per day for 3 days, or 100 mg per day for 14 days, all fasting state) in Part 2. For all cohorts, the daily dose was split into 2 equal doses, administered 12 hours apart (i.e., BID; Q12hr) and Compound and placebo were administered in capsule dosage form. For Cohort 1, subjects received oral administration of Compound (N=10) or matching placebo (N=2), 50 mg BID for 6 days with a final dose of 50 mg on the morning of day 7. For Cohort 2, subjects received oral administration of Compound (N=10) or matching placebo (N=2), 100 mg BID for 6 days with a final dose of 100 mg on the morning of day 7. For Cohort 3, subjects received oral administration of Compound (N=10) or matching placebo (N=2), 50 mg BID for 13 days with a final dose of 50 mg on the morning of day 14. For Cohort 4, subjects received oral administration of Compound (N=10) or matching placebo (N=2), 200 mg BID for 2 days with a final dose of 200 mg on the morning of day 3. For Cohort 5, subjects received oral administration of Compound (N=10) or matching placebo (N=2), 400 mg BID for 2 days with a final dose of 400 mg on the morning of day 3. All subjects completed their respective dosing regimens except for Cohort 3, where 7 of the 12 subjects completed the dosing regimen. Those subjects that discontinued early and were randomized to receive Compound were excluded from the Day 14 PK analysis.

All enrolled subjects in each Cohort had PK samples collected through at least 48 hours after dosing on Day 1 and all enrolled subject that completed their dosing regimen had PK samples collected through at least 24 hours after dosing on the final day of dosing. After dosing on Day 1, plasma concentrations of Compound were quantifiable through 12 hours in all subjects in all Cohorts and after administration of the final dose, plasma concentrations of Compound were quantifiable through at least 24 hours in all subjects in all Cohorts. Plasma concentrations of Compound were quantifiable before administration of the final dose in all subject in all Cohorts. For Day 1 PK data, only data through 12 hours is reported, as all data after the second dose at 12 hours is reflective of accumulation.

Table 2 provides a summary of key pharmacokinetic parameters of Compound in healthy subjects following oral administration of Compound every 12 hours as described above for Part 2.

Compound administered as multiple ascending oral doses for 3, 7, or 14 days in Part 2 was safe and generally well tolerated in all subjects in all Cohorts (with the exception of Cohort 3) with no serious adverse events, no clinically significant changes is safety laboratory parameters and no dose-dependent safety signals were observed. There were 5 study discontinuations (all in Cohort 3). Benign rash in a number of subjects was observed and was self-limited and resolved within a median of 5 days of onset. Incidence of skin rashes had no dose response relationship.

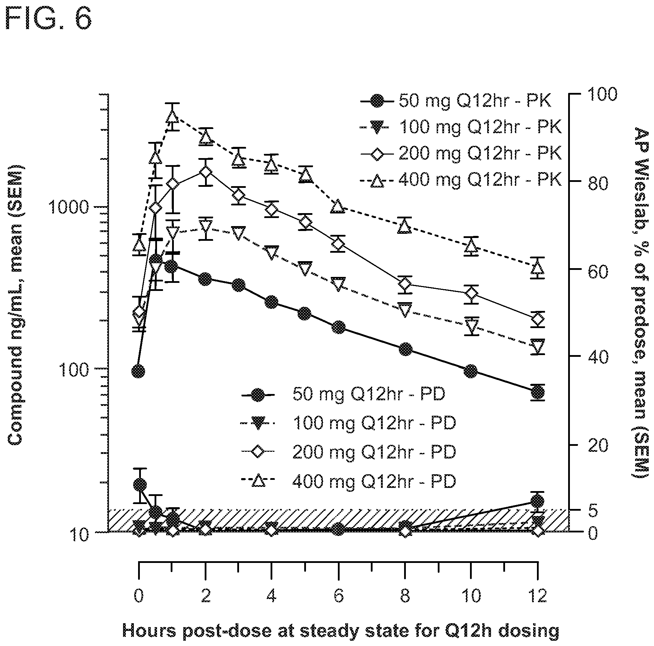

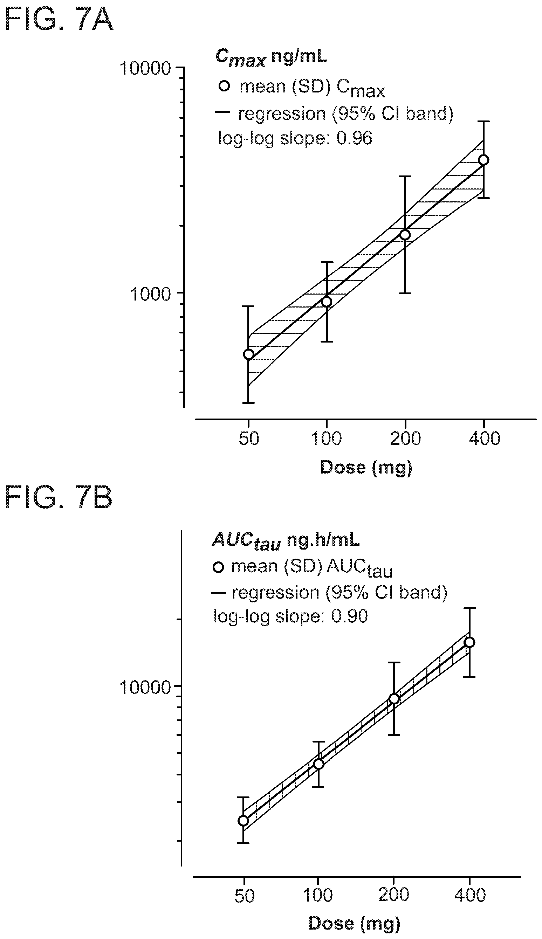

Compound exposure for these multiple ascending doses was well characterized over the sampling period (Figure 6; Table 2) and approximately linear and dose-proportional (Figures 7A-B). Mean Compound concentration at 12 hours post dose was within or above the anticipated threshold of 8 to 10 times the factor D EC₅₀ for multiple ascending doses ≥ 200 mg/day. Compound exposure was approximately 50% higher in Cohort 3 as compared to Cohort 1 both on Day 1 and at steady state, despite the same dose being administered in each Cohort. Following an investigation no bias or process error was identified and the increase in exposure was attributed to be due to inherent pharmacokinetic variability and small sample size. Increase in exposure to Compound was mild in all Cohorts (see accumulation ratios Day 7/Day 1 in Table 2). Based on data for these five cohorts, Tₘₐₓ does not appear to be impacted by multiple dosing. Of note, the accumulation ratio in Cohorts 4 and 5 was highly consistent with those of Cohorts 1 through 3, suggesting that steady state is reached following 5 doses of Compound every 12 hours (Table 2).

**Table 2: Cumulative geometric mean (CV%) of key pharmacokinetic parameters of Compound in healthy subjects following oral administration of Compound in capsules every 12 hours.**

| | **Cohort 1, 1^{st} Dose** | **Cohort 2, 1^{st} Dose** | **Cohort 3, 1^{st} Dose** | **Cohort 4, 1^{st} Dose** | **Cohort 5, 1^{st} Dose** |
|---|---|---|---|---|---|
| **Regimen** | **50 mg BID** | **100 mg BID** | **50 mg BID** | **200 mg BID** | **400 mg BID** |
| **Tₘₐₓ^{a}** | 2 (0.5-3) | 2 (0.5-3) | 1 (0.5-2) | 2 (1-6) | 1.5 (0.4-4) |
| **Cₘₐₓ (ng/mL)** | 405 (44.9) | 901 (32.1) | 602 (30.7) | 1243 (45.9) | 3137 (41.5) |
| **C₁₂^{b} (ng/mL)** | 39.4 (44.2) | 67.8 (31.1) | 49.0 (36.9) | 125.8 (47.2) | 223.2 (36.4) |
| **AUC₀₋₁₂ (ng*h/mL)** | 1662 (42.3) | 3154 (19.8) | 2180 (30.9) | 5873 (38.1) | 11479 (23.6) |
| **AUC₀₋₂₄ (ng*h/mL)** | 3141 (33.4) | 6151 (18.5) | 4095 (30.4) | 11952 (32.8) | 25193 (31.2) |
| | | | | | |

| | **Cohort 1, Day 7** | **Cohort 2, Day 7** | **Cohort 3, Day 14** | **Cohort 4, Day 3** | **Cohort 5, Day 3** |
|---|---|---|---|---|---|
| **Regimen** | **50 mg BID** | **100 mg BID** | **50 mg BID** | **200 mg BID** | **400 mg BID** |
| **Tₘₐₓ^{a}** | 1.5 (0.5-3) | 2.5 (1-3) | 1 (0.5-3) | 2 (0.5-5) | 1 (0.5-5) |
| **Cₘₐₓ (ng/mL)** | 539 (52.0) | 918 (41.9) | 883 (19.6) | 1817 (65.4) | 3958 (42.0) |
| **C₁₂^{b} (ng/mL)** | 73.2 (34.4) | 136.4 (30.8) | 84.3 (28.1) | 222.1 (31.4) | 431.1 (44.0) |
| **T_{1/2}^{c}** | 6.5 (29.8) | 5.8 (22.3) | 5.8 (9.6) | 6.2 (14.1) | 6.9 (23.7) |
| **AUCₜₐᵤ (ng*h/mL)** | 2485 (24.8) | 4460 (23.9) | 3818 (11.1) | 8809 (39.2) | 15918 (37.1) |
| **AUC_{0-inf} (ng*h/mL)** | 3449 (27.4) | 5968 (29.8) | 4863 (13.2) | 11320 (35.9) | 20394 (40.9) |
| **Accumulation Ratio^{d}** | 1.50 (34.2) | 1.41 (20.5) | 1.48 (21.6) | 1.50 (18.8) | 1.39 (18.6) |

| | | | | | |
|---|---|---|---|---|---|
| a. Median and range. b. Arithmetic mean and CV. c. Calculated for Cohorts 1 and 2 using best-fit slope of all available PK time points and for Cohorts 3-5 using elimination rate constant estimated via slope of Compound concentration vs time profiles from 8 to 24 hours. d. Calculated as ratio of steady-state AUCₜₐᵤ to Day 1 AUC₀₋₁₂ | | | | | |

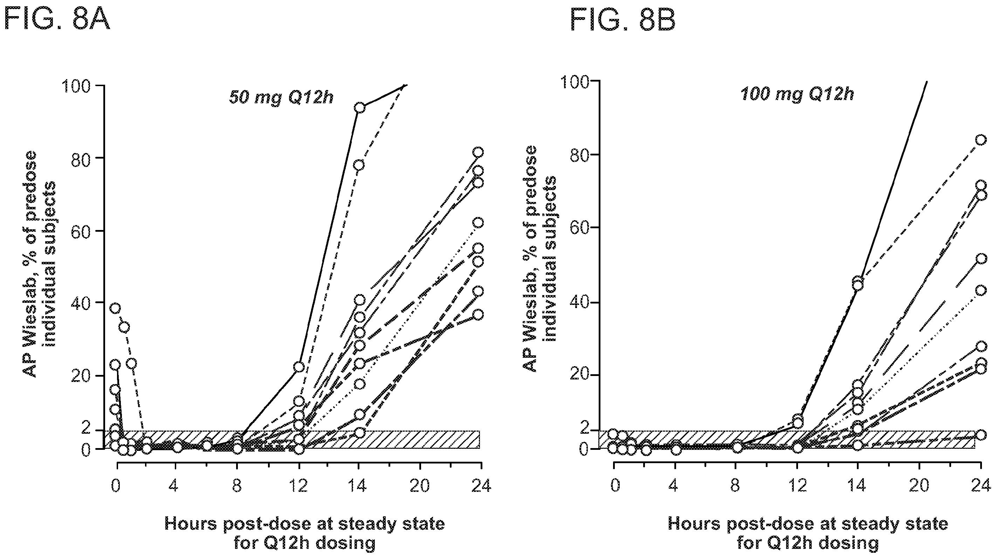

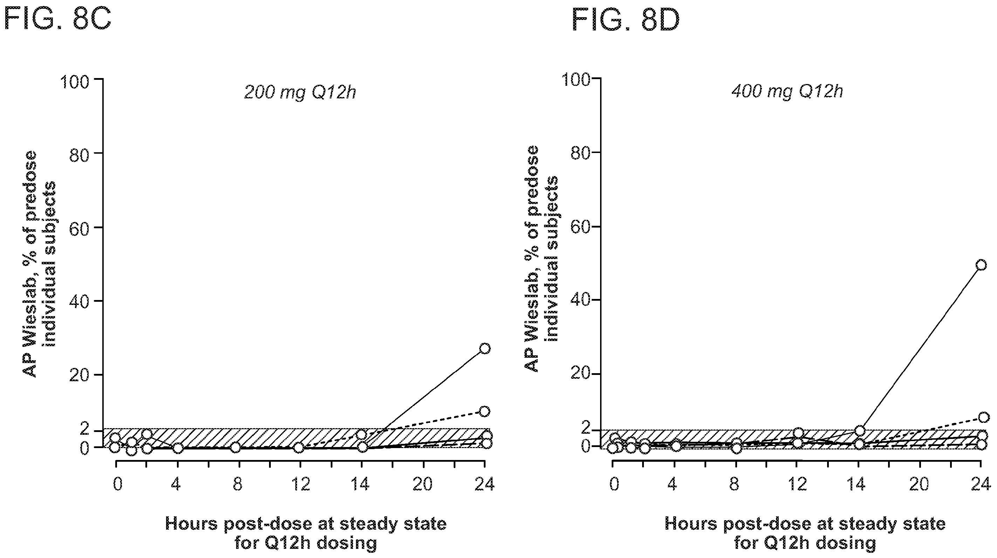

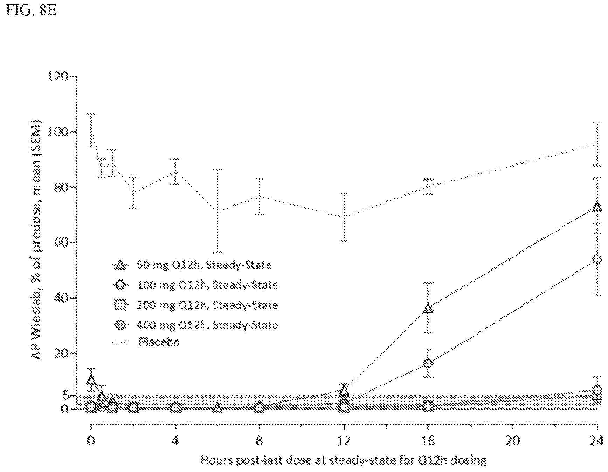

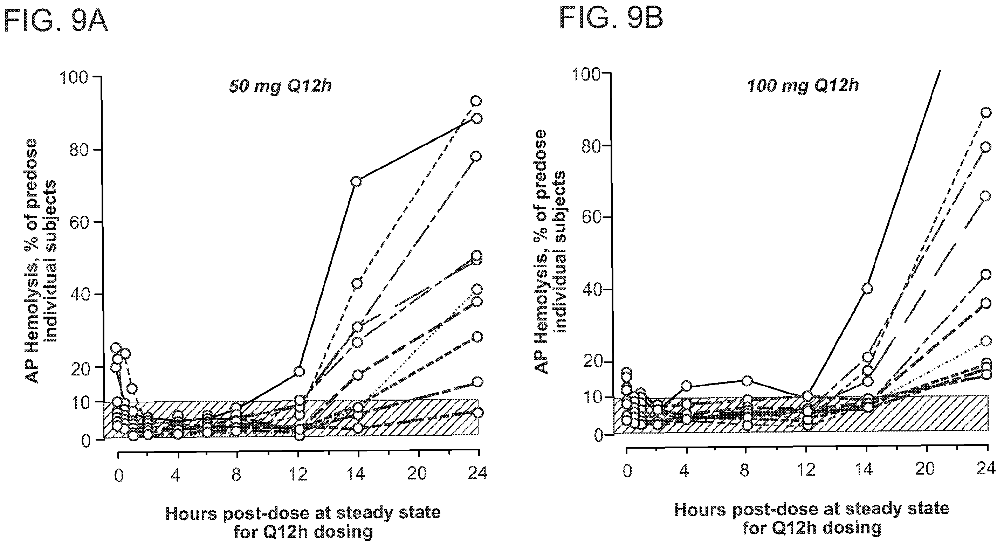

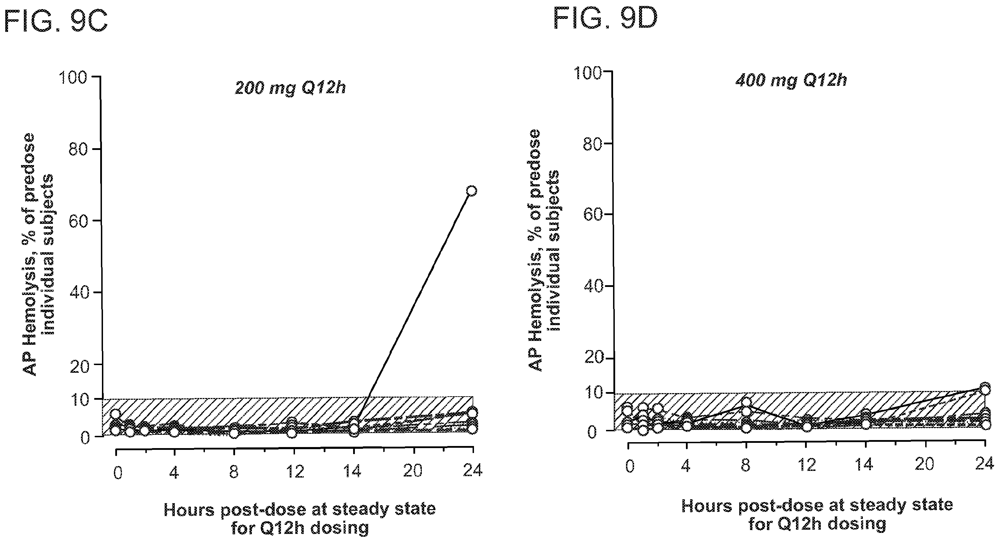

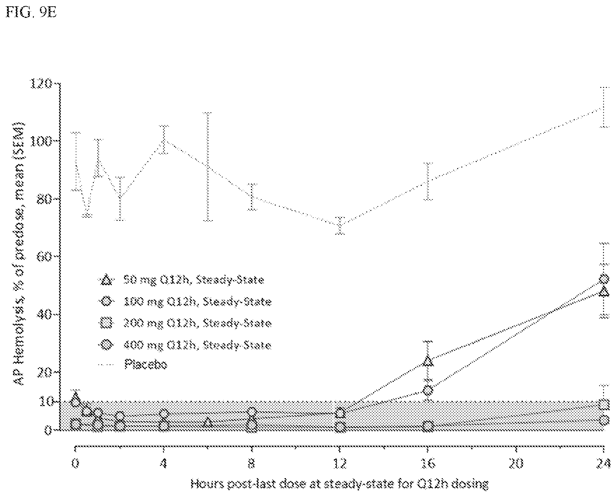

Figures 8A-D show individual subject values for the inhibition of AP Wieslab activity for Cohorts 1, 2, 4, and 5, respectively, of Part 2 and demonstrates dose-dependent suppression. Greater than 90% suppression of AP Wieslab activity was sustained for 12 hours after administration of the final dose on day 7 in Cohort 1 (93.2% ± 6.8% inhibition) and Cohort 2 (97.9% ± 3.0% inhibition) (Figures 8A-B). Greater than 90% suppression of AP Wieslab activity was sustained for over 24 hours after administration of the final dose on day 3 in Cohort 4 (95.0% ± 8.2% inhibition) and Cohort 5 (93.3% ± 15.3% inhibition) (Figures 8C-D). Figure 8E shows the arithmetic mean (± SEM) of AP Wieslab activity as a percent of baseline for all subjects in Cohorts 1, 2, 4, and 5 of Part 2 at steady state of the Compound. Figures 9A-D show individual subject values for the inhibition of AP hemolysis for Cohorts 1, 2, 4, and 5, respectively, of Part 2 and demonstrates dose-dependent suppression. Greater than 90% suppression of AP hemolysis was sustained for 12 hours after administration of the final dose on day 7 in Cohort 1 (93.8% ± 5.3% inhibition) and Cohort 2 (94.1% ± 2.6% inhibition) (Figures 9A-B). Greater than 90% suppression of AP hemolysis was sustained for over 24 hours after administration of the final dose on day 3 in Cohort 4 (91.1% ± 20.5% inhibition) and Cohort 5 (96.4% ± 3.8% inhibition) (Figures 9C-D). Figure 9E shows the arithmetic mean (± SEM) of AP Hemolysis activity as a percent of baseline for all subjects in Cohorts 1, 2, 4, and 5 of Part 2 at steady state of the Compound. Figures 8E and 9E shows ≥ 90% suppression of AP activity was observed for the multiple ascending doses of Cohorts 1, 2, 4, and 5 of Part 2 for 12 hours following administration, with the duration and magnitude of suppression increasing with dose. The inhibition values shown for Figures 8A-D and 9A-D are compared to pre-dose values and are reported as mean values (± SD of the mean) derived from all subjects in each Cohort.

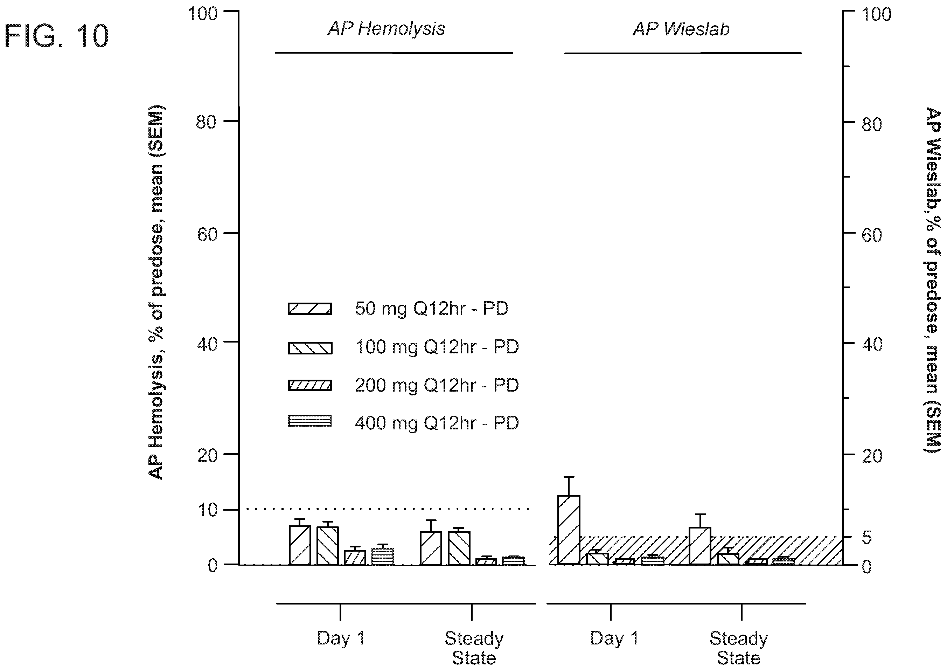

Figure 10 shows PD profiles (AP hemolysis and AP Wieslab activity) of the Compound in Cohorts 1, 2, 4, and 5 on Day 1 (24 hours) and steady state PK troughs. Significant inhibition of AP hemolysis and AP Wieslab activity was observed in Cohorts 1, 2, 4, and 5, with Cohorts 1, 2, 4, and 5 showing greater than 90% inhibition of AP induced hemolysis at Day 1 and steady-stated PK troughs and Cohorts 2, 4, and 5 showing greater than 95% inhibition of AP Wieslab activity at Day 1 and steady-stated PK troughs. In Cohorts 4 and 5, AP activity is blocked by greater than 98% in both the AP hemolysis and AP Wieslab assays throughout the dosing interval at steady state. Importantly, the higher doses provide more consistent PD effects, which is important in PNH treatment.

PK/PD modeling of AP hemolysis and AP Wieslab data demonstrates a clear concentration-response relationship, with estimated EC₅₀ values between 21.7 and 40.7 nM. These estimates are consistent with the *in vitro* IC₅₀ for inhibition of proteolytic activity against C3bB of 28.1 nM.

In summary, for Part 2 Compound administered as multiple ascending oral doses from 100 to 800 mg/day for 3 to 14 days was safe and generally well tolerated in all subjects with no Compound-related safety concerns of note. Furthermore, Compound administered as multiple ascending oral doses from 100 to 800 mg/day for 3 to 14 days demonstrated clinically beneficial and dose-dependent inhibition of the AP activity.

### Part 3 - Multiple Ascending Oral Doses of Compound in Subjects with PNH

Part 3 is currently ongoing. Subjects with PNH are recruited into two groups: Part 3A, which includes subjects with PNH who are naive to C5-inhibitor treatment (such as eculizumab or ravulizumab), and Part 3B, which includes subjects with PNH who are currently being treated with a C5-inhibitor (such as eculizumab or ravulizumab) and are poor responders to C5-inhibitor therapy with Compound added to existing PNH treatments. Subjects may be enrolled concurrently into Parts 3A and 3B. Up to four sequential cohorts for Parts 3A and 3B may be enrolled, each using a forced titration design for 28 days. Four subjects have been enrolled in Cohort 1 of Part 3A; Cohort 2 of Part 3A is currently enrolling subjects. Dosing regimens in Period 1 and 2 of Cohorts 3 and 4 may be the same.

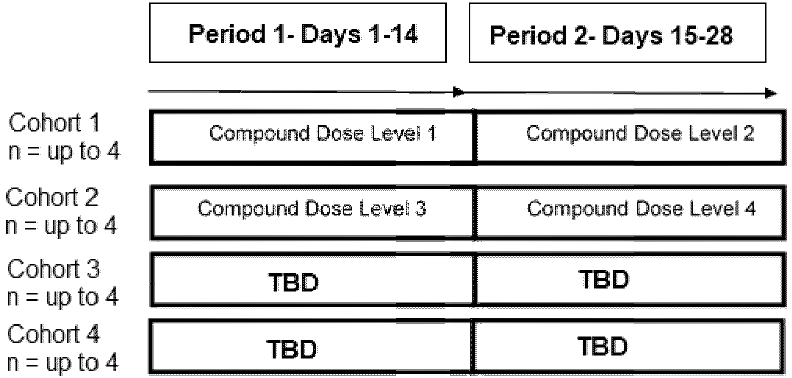

Additional Cohorts in Parts 3A and 3B may be enrolled to test higher dosing levels, 1 time per day (i.e., QD) administration, and/or administration of a single dose (administered either QD or BID) throughout the 28 days dosing period.

Subjects in Part 3A are treated with a 28-day course of study drug (Compound or placebo) administered orally in capsule dose form. For Cohorts 1 and 2 of Part 3A, the daily dose is split into 2 equal doses administered 12 hours apart (i.e., BID or Q12hr). After 14 days of dosing at 50 mg BID, subjects in Part 3A Cohort 1 were escalated to a dose of 100 mg BID for an additional 14 days of dosing. Dosing in Part 3A Cohort 2 will start after independent data review of the data collected in Cohort 1. Dosing in Part 3A Cohort 2 will be as described for Cohort 1, except that the dose of Compound will be increased to 200 mg BID (days 1-14) and 400 mg BID (days 15-28). Dosing in Part 3B Cohort 1 may be initiated at 200 mg BID for 14 days and increased to 400 mg BID for an additional 14 days of dosing; dosing in Part 3B Cohort 2 may be further escalated. After 28 days of total dosing, subjects with positive response to treatment may continue to receive treatment for up to a total of 48 weeks, depending on responsiveness to treatment. Subjects continuing to receive Compound in the 48- week extension (i.e., subjects with positive response to treatment) will be monitored for safety and PD profiles as described herein. The 48-week extension includes the option for individual dose titrations for incomplete clinical benefit. Safety and tolerability of the Compound are evaluated as described for Part 1 and 2 herein, and plasma concentrations and PD effects of the Compound are determined as described in Parts 1 and 2 herein.

The primary endpoints of Part 3 are safety and tolerability based on laboratory and examination findings. Secondary endpoints for Part 3 include the identification of a therapeutically active dose regimen in PNH patients, determination of pharmacokinetic parameters (including Cₘₐₓ, Tₘₐₓ, T_{1/2}, and AUCₜₐᵤ) and pharmacodynamics profiles (including plasma factor Bb, PNH clone size, number of blood transfusions, lactate dehydrogenase activity, hemoglobin levels, bilirubin levels, absolute reticulocyte count, and haptoglobin levels) in PNH patients.

Initial results from four subjects in Cohort 1, Part 3A have been obtained. Compound administered as ascending oral doses of 50 mg BID for 14 days followed by 100 mg BID for an additional 14 days was safe and generally well tolerated for the four subjects of Cohort 1 in Part 3A. There were no study discontinuations, no serious adverse events, and no clinically significant changes is safety laboratory parameters. All four subjects reported mild headache 1-3 days after administration of Compound. All headaches were mild in severity and resolved spontaneously. No rashes were observed for the four subjects in Part 3A Cohort 1.

Table 3 below shows the baseline characteristics for the four subjects in Part 3A Cohort 1. As can be seen in Table 3, all four subjects were seriously ill with PNH. Subject 1 had a previous cerebral vein thrombosis from PNH, subject 2 required multiple red blood cell (RBC) transfusions in the year prior to screening and subject 3 was diagnosed with aplastic anemia-PNH.

Consistent with other PNH studies, subjects 1-4 showed variable pre-treatment degrees of hemolysis and anemia. Among the four subjects of Part 3A, Cohort 1, the lactate dehydrogenase (LDH) level, a sensitive marker of hemolysis, ranged over 800 to over 2400 IU/L, or 3.7 to 11 times the upper limit of normal (ULN). The degree of anemia was severe, with hemoglobin of 6.0 to 10.7 g/dL. All four subjects had elevated reticulocyte counts (130 to 285 x 10³ cell/µL), reflecting the bone marrow working harder to increase hemoglobin levels.

**Table 3: Baseline characteristics for the subjects enrolled in Part 3A Cohort 1**

| **Pre-treatment Characteristic*** | **Subject 1** | **Subject 2** | **Subject 3** | **Subject 4** |
|---|---|---|---|---|
| PNH duration, years | 8 | 4 | 3 | 5 |
| History of aplastic anemia | No | No | Yes | No |
| History of thrombosis | Yes | No | No | No |
| LDH, IU/L | 2205 | 2497 | 835 | 1719 |
| LDH X ULN | 9.8 | 11.0 | 3.7 | 6.9 |
| Hemoglobin, g/dL | 8.2 | 7.0 | 6.0 | 10.7 |
| Reticulocytes, 10³ cell/µL | 220 | 285 | 130 | 200 |
| Total bilirubin, mg/dL | 3.33 | 1.47 | 1.12 | 0.61 |
| PNH type III erythrocyte clone size, % | 89 | 41 | 49 | 49 |
| Units of RBC transfused in 52 weeks prior to screening | 0 | 13 | 0 | 2 |
| Units of RBC transfused in 12 weeks prior to screening | 0 | 2 | 0 | 0 |
| * Laboratory values for LDH, reticulocyte count, total bilirubin, and PNH type III erythrocyte clone size pre-administration (pre-Rx) are the average of all available screening and baseline results. For hemoglobin, the pre-Rx value was the last available data prior to Day 1. | | | | |

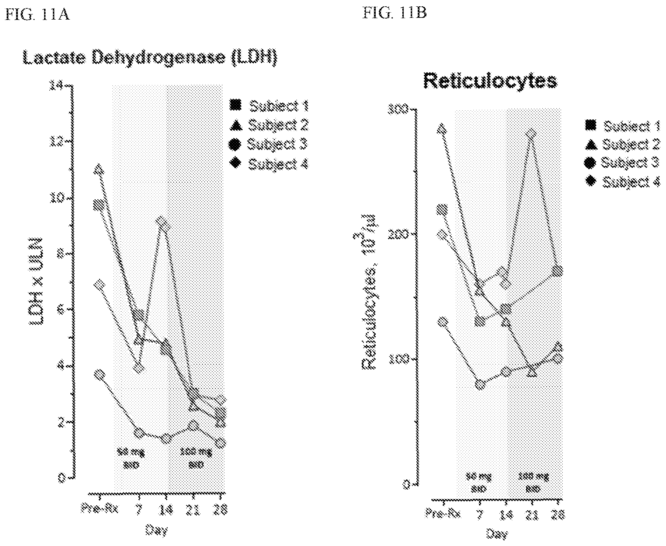

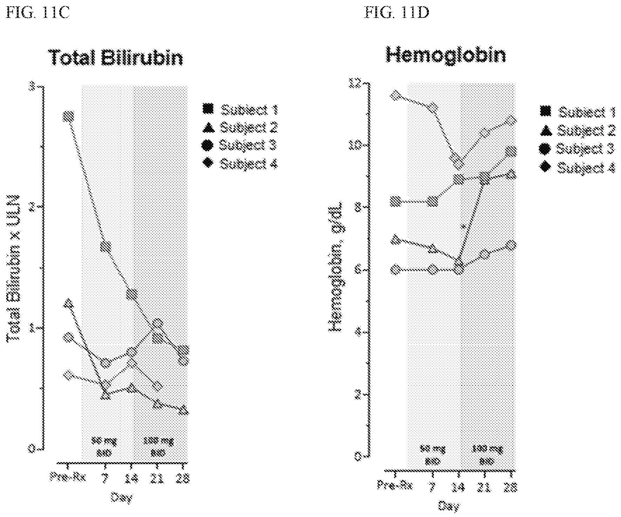

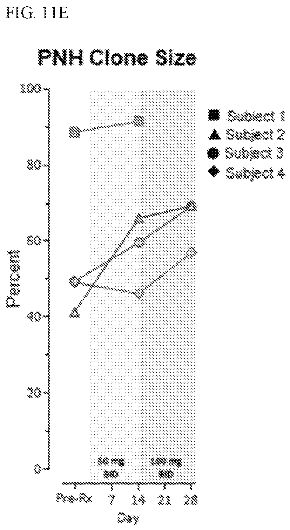

Figures 11A-11E show individual subject PD profiles of the Compound from 3 subjects through 28 days of administration of Compound as described above for Cohort 1 of Part 3A. All four subjects showed clinically meaningful and dose dependent reduction across all hemolysis biomarkers. Decreases in LDH (4/4 subjects) and reticulocytes (3/4 subjects), and bilirubin (2/2 subjects with elevated pretreatment values) were observed following 28 days of dosing (Figures 11A-11B). Total bilirubin, another marker of hemolysis in PNH, was elevated in 2 subjects at baseline and normalized following 28 days of dosing (Figure 11C). Previous studies of complement inhibitors have shown it takes approximately 8 weeks to see stabilization of hemoglobin levels with optimized doses. Following 28 days of dosing, Hemoglobin levels were already increasing at the lowest doses of Compound (3/4 subjects) (Figure 11D). PNH type III erythrocyte clone size also increased or was maintained in all four subjects following 28 days of dosing (Figure 11E). As show in in Table 3, subject 2 was dependent on RBC transfusions, with a pre-administration hemoglobin level of 7.0 g/dL. Following a 2-unit RBC transfusion on Day 15, subject 2 has been transfusion-free for 6 weeks and hemoglobin levels have risen from 8.9 g/dL post-transfusion on Day 15 to 11.1 g/dL at week 8 (while continuing to receive Compound at a dose of 100 mg BID).

Subject 4 showed an initial response to treatment with Compound, followed by worsening indicators of hemolysis temporally associated with an upper respiratory tract infection (URTI; onset on Day 13). With an increase in dose to 100 mg BID and resolution of the URTI, LDH level and reticulocyte count fell and hemoglobin levels rose.

Three of four subjects reported PNH-associated symptoms, including fatigue, erectile dysfunction, headache and abdominal pain, prior to enrollment. In all cases, symptoms resolved by Day 21 of Compound administration.

The results in Figures 11A-E show improvement in key biomarkers of hemolysis in PNH and demonstrate the clinical utility of the Compound in treating or preventing PNH when administered as described above.

All four subjects continued into the extension. In Subjects 2 to 4 currently enrolled in the extension, the dose of Compound has been increased to 200 mg BID, a dose regimen that demonstrated ≥ 98% AP inhibition in healthy subjects with substantially less inter-subject variability than that observed with lower doses. Initial data from these three subjects showed that after 14 days of 200 mg BID, LDH values were 1.47 x ULN in Subjects 3 and 4 (4.4 x ULN in Subject 2) and hemoglobin levels were 1 mg/dL and 1.6 mg/dL higher than after 14 days of 100 mg BID in Subjects 2 and 3, respectively (with data unavailable for Subject 4).

In summary, for Cohort 1 of Part 3A Compound administered as ascending oral doses of 50 mg BID for 14 days followed by 100 mg BID for an additional 14 days was safe and generally well tolerated in all subjects with no Compound-related safety concerns of note. Furthermore, Compound administered as ascending oral doses of 50 mg BID for 14 days followed by 100 mg BID for an additional 14 days demonstrated clinically beneficial effects on key biomarkers of hemolysis in PNH.

### CONCLUSIONS:

Oral dosing with compounds for use in the invention, potent inhibitors of human complement factor D, was safe and generally well tolerated in all subjects of Parts 1 to 3 that have completed treatment, with no Compound-related safety concerns of note. Oral dosing with compounds for use in the invention demonstrated linear and dose-proportional exposure, with PD parameters correlating well with the observed PK and showing rapid, sustained, and dose-dependent inhibition of AP complement activity in Parts 1 and 2. Oral dosing with compounds for use in the invention in Part 3 showed clinically beneficial effects on physiological parameters effected by PNH. The present disclosure demonstrates clinical utility of the Compound in treating or preventing PNH when administered as described herein.

## Claims

1. A compound selected from: 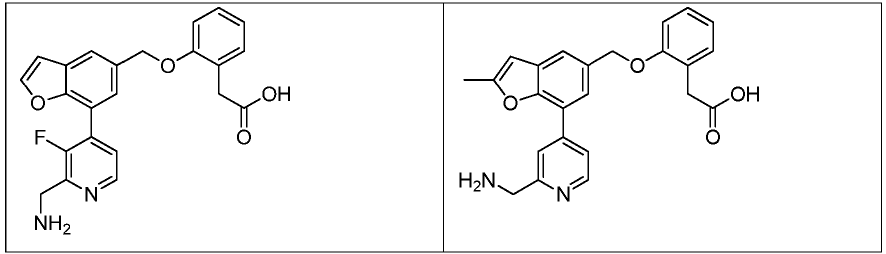
, or a pharmaceutically acceptable salt thereof, for use in a method of treating or preventing paroxysmal nocturnal haemoglobinuria (PNH), the method comprising orally administering to a patient in need thereof a therapeutically effective amount of the compound, or a pharmaceutically acceptable salt thereof;
wherein the PNH is **characterized by** one or more of hemolytic anemia, thrombosis, and impaired bone marrow function.

2. The compound for use according to claim 1, wherein the therapeutically effective amount is 1 mg to 1500 mg of the compound or a pharmaceutically acceptable salt thereof;
or wherein the therapeutically effective amount is 1 mg to 1200 mg of the compound or a pharmaceutically acceptable salt thereof;
or wherein the therapeutically effective amount is 1 to 1000 mg of the compound or a pharmaceutically acceptable salt thereof;
or wherein the therapeutically effective amount is 1 to 800 mg of the compound or a pharmaceutically acceptable salt thereof;
or wherein the therapeutically effective amount is 1 to 600 mg of the compound or a pharmaceutically acceptable salt thereof;
or wherein the therapeutically effective is 1 to 400 mg of the compound or a pharmaceutically acceptable salt thereof;
or wherein the therapeutically effective amount is 1 to 300 mg of the compound or a pharmaceutically acceptable salt thereof;
or wherein the therapeutically effective amount is 1 to 200 mg of the compound or a pharmaceutically acceptable salt thereof;
or wherein the therapeutically effective amount is 1 to 100 mg of the compound or a pharmaceutically acceptable salt thereof;
or wherein the therapeutically effective amount is 10 mg of the compound or a pharmaceutically acceptable salt thereof;
or wherein the therapeutically effective amount is 30 mg of the compound or a pharmaceutically acceptable salt thereof;
or wherein the therapeutically effective amount is 100 mg of the compound or a pharmaceutically acceptable salt thereof;
or wherein the therapeutically effective amount is 200 mg of the compound or a pharmaceutically acceptable salt thereof;
or wherein the therapeutically effective amount is 300 mg of the compound or a pharmaceutically acceptable salt thereof;
or wherein the therapeutically effective amount is 400 mg of the compound or a pharmaceutically acceptable salt thereof;
or wherein the therapeutically effective amount is 600 mg of the compound or a pharmaceutically acceptable salt thereof;
or wherein the therapeutically effective amount is 800 mg of the compound or a pharmaceutically acceptable salt thereof;
or wherein the therapeutically effective amount is 1000 mg of the compound or a pharmaceutically acceptable salt thereof;
or wherein the therapeutically effective amount is 1200 mg of the compound or a pharmaceutically acceptable salt thereof.

3. The compound for use according to claim 1 or 2, wherein the therapeutically effective amount is 1 to 1000 mg of the compound or a pharmaceutically acceptable salt thereof.

4. The compound for use according to claim 1 or 2, wherein the therapeutically effective amount is 1 to 800 mg of the compound or a pharmaceutically acceptable salt thereof.

5. The compound for use according to claim 1 or 2, wherein the therapeutically effective amount is 1 to 600 mg of the compound or a pharmaceutically acceptable salt thereof.

6. The compound for use according to claim 1 or 2, wherein the therapeutically effective amount is 1 to 400 mg of the compound or a pharmaceutically acceptable salt thereof.

7. The compound for use according to claim 1 or 2, wherein the therapeutically effective amount is 200 mg.

8. The compound for use according to claim 1 or 2, wherein the therapeutically effective amount is 300 mg.

9. The compound for use according to claim 1 or 2, wherein the therapeutically effective amount is 600 mg.

10. The compound for use according to any one of claims 1-9, wherein the compound or a pharmaceutically acceptable salt thereof is administered once daily.

11. The compound for use according to any one of claims 1-9, wherein the compound or a pharmaceutically acceptable salt thereof is administered twice daily.

12. The compound for use according to any one of claims 1-9, wherein the compound or a pharmaceutically acceptable salt thereof is administered three times daily.

13. The compound for use according to any one of claims 1-12, wherein the compound is 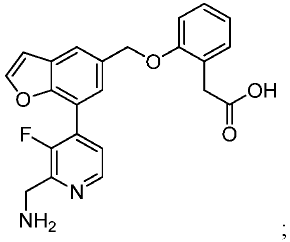 or a pharmaceutically acceptable salt thereof.

14. The compound for use according to any one of claims 1-12, wherein the compound is 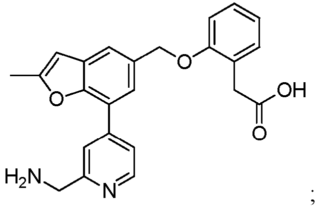 or a pharmaceutically acceptable salt thereof.

## Patentansprüche

1. Verbindung ausgewählt aus: 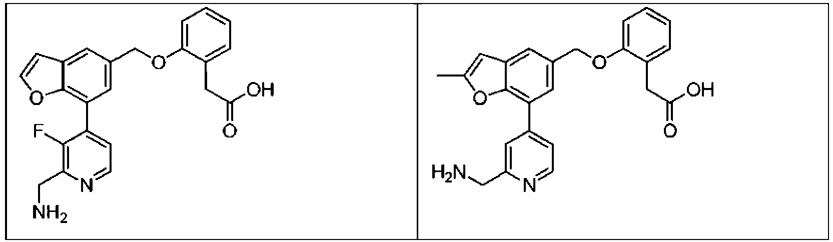
oder einem pharmazeutisch verträglichen Salz davon, zur Verwendung in einem Verfahren zum Behandeln oder Verhindern von paroxysmaler nächtlicher Hämoglobinurie (PNH), wobei das Verfahren orales Verabreichen einer therapeutisch wirksamen Menge der Verbindung oder eines pharmazeutisch verträglichen Salzes davon an einen Patienten, der dessen bedarf, umfasst;
wobei die PNH **gekennzeichnet ist durch** eine oder mehrere von hämolytischer Anämie, Thrombose und beeinträchtigter Knochenmarkfunktion.

2. Verbindung zur Verwendung nach Anspruch 1, wobei die therapeutisch wirksame Menge 1 mg bis 1500 mg der Verbindung oder eines pharmazeutisch verträglichen Salzes davon beträgt;
oder wobei die therapeutisch wirksame Menge 1 mg bis 1200 mg der Verbindung oder eines pharmazeutisch verträglichen Salzes davon beträgt;
oder wobei die therapeutisch wirksame Menge 1 mg bis 1000 mg der Verbindung oder eines pharmazeutisch verträglichen Salzes davon beträgt;
oder wobei die therapeutisch wirksame Menge 1 mg bis 800 mg der Verbindung oder eines pharmazeutisch verträglichen Salzes davon beträgt;
oder wobei die therapeutisch wirksame Menge 1 mg bis 600 mg der Verbindung oder eines pharmazeutisch verträglichen Salzes davon beträgt;
oder wobei die therapeutisch wirksame Menge 1 mg bis 400 mg der Verbindung oder eines pharmazeutisch verträglichen Salzes davon beträgt;
oder wobei die therapeutisch wirksame Menge 1 mg bis 300 mg der Verbindung oder eines pharmazeutisch verträglichen Salzes davon beträgt;
oder wobei die therapeutisch wirksame Menge 1 mg bis 200 mg der Verbindung oder eines pharmazeutisch verträglichen Salzes davon beträgt;
oder wobei die therapeutisch wirksame Menge 1 mg bis 100 mg der Verbindung oder eines pharmazeutisch verträglichen Salzes davon beträgt;
oder wobei die therapeutisch wirksame Menge 10 mg der Verbindung oder eines pharmazeutisch verträglichen Salzes davon beträgt;
oder wobei die therapeutisch wirksame Menge 30 mg der Verbindung oder eines pharmazeutisch verträglichen Salzes davon beträgt;
oder wobei die therapeutisch wirksame Menge 100 mg der Verbindung oder eines pharmazeutisch verträglichen Salzes davon beträgt;
oder wobei die therapeutisch wirksame Menge 200 mg der Verbindung oder eines pharmazeutisch verträglichen Salzes davon beträgt;
oder wobei die therapeutisch wirksame Menge 300 mg der Verbindung oder eines pharmazeutisch verträglichen Salzes davon beträgt;
oder wobei die therapeutisch wirksame Menge 400 mg der Verbindung oder eines pharmazeutisch verträglichen Salzes davon beträgt;
oder wobei die therapeutisch wirksame Menge 600 mg der Verbindung oder eines pharmazeutisch verträglichen Salzes davon beträgt;
oder wobei die therapeutisch wirksame Menge 800 mg der Verbindung oder eines pharmazeutisch verträglichen Salzes davon beträgt;
oder wobei die therapeutisch wirksame Menge 1000 mg der Verbindung oder eines pharmazeutisch verträglichen Salzes davon beträgt;
oder wobei die therapeutisch wirksame Menge 1200 mg der Verbindung oder eines pharmazeutisch verträglichen Salzes davon beträgt.

3. Verbindung zur Verwendung nach Anspruch 1 oder 2, wobei die therapeutisch wirksame Menge 1 bis 1000 mg der Verbindung oder eines pharmazeutisch verträglichen Salzes davon beträgt.

4. Verbindung zur Verwendung nach Anspruch 1 oder 2, wobei die therapeutisch wirksame Menge 1 bis 800 mg der Verbindung oder eines pharmazeutisch verträglichen Salzes davon beträgt.

5. Verbindung zur Verwendung nach Anspruch 1 oder 2, wobei die therapeutisch wirksame Menge 1 bis 600 mg der Verbindung oder eines pharmazeutisch verträglichen Salzes davon beträgt.

6. Verbindung zur Verwendung nach Anspruch 1 oder 2, wobei die therapeutisch wirksame Menge 1 bis 400 mg der Verbindung oder eines pharmazeutisch verträglichen Salzes davon beträgt.

7. Verbindung zur Verwendung nach Anspruch 1 oder 2, wobei die therapeutisch wirksame Menge 200 mg beträgt.

8. Verbindung zur Verwendung nach Anspruch 1 oder 2, wobei die therapeutisch wirksame Menge 300 mg beträgt.

9. Verbindung zur Verwendung nach Anspruch 1 oder 2, wobei die therapeutisch wirksame Menge 600 mg beträgt.

10. Verbindung zur Verwendung nach einem der Ansprüche 1 bis 9, wobei die Verbindung oder ein pharmazeutisch verträgliches Salz davon einmal täglich verabreicht wird.

11. Verbindung zur Verwendung nach einem der Ansprüche 1 bis 9, wobei die Verbindung oder ein pharmazeutisch verträgliches Salz davon zweimal täglich verabreicht wird.

12. Verbindung zur Verwendung nach einem der Ansprüche 1 bis 9, wobei die Verbindung oder ein pharmazeutisch verträgliches Salz davon dreimal täglich verabreicht wird.

13. Verbindung zur Verwendung nach einem der Ansprüche 1 bis 12, wobei die Verbindung Folgendes ist: 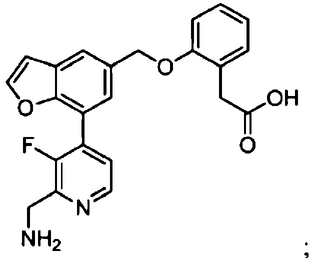 oder ein pharmazeutisch verträgliches Salz davon.

14. Verbindung zur Verwendung nach einem der Ansprüche 1 bis 12, wobei die Verbindung Folgendes ist: 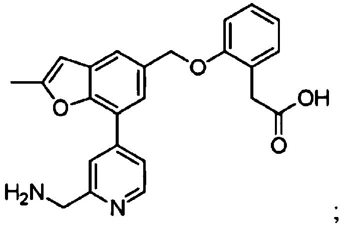 oder ein pharmazeutisch verträgliches Salz davon.

## Revendications

1. Composé choisi parmi : 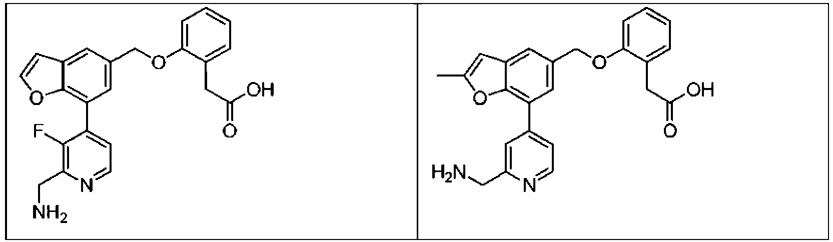
ou un sel pharmaceutiquement acceptable de celui-ci, destiné à être utilisé dans un procédé de traitement ou de prévention de l'hémoglobinurie paroxystique nocturne (HPN), le procédé comprenant l'administration orale à un patient qui en a besoin d'une quantité thérapeutiquement efficace du composé, ou d'un sel pharmaceutiquement acceptable de celui-ci ;
dans lequel l'HPN est **caractérisée par** une ou plusieurs parmi l'anémie hémolytique, la thrombose et la détérioration de la fonction de la moelle osseuse.

2. Composé destiné à être utilisé selon la revendication 1, dans lequel la quantité thérapeutiquement efficace est comprise entre 1 mg et 1500 mg du composé ou d'un sel pharmaceutiquement acceptable de celui-ci ;
ou dans lequel la quantité thérapeutiquement efficace est comprise entre 1 mg et 1200 mg du composé ou d'un sel pharmaceutiquement acceptable de celui-ci ;
ou dans lequel la quantité thérapeutiquement efficace est comprise entre 1 et 1000 mg du composé ou d'un sel pharmaceutiquement acceptable de celui-ci ;
ou dans lequel la quantité thérapeutiquement efficace est comprise entre 1 et 800 mg du composé ou d'un sel pharmaceutiquement acceptable de celui-ci ;
ou dans lequel la quantité thérapeutiquement efficace est comprise entre 1 et 600 mg du composé ou d'un sel pharmaceutiquement acceptable de celui-ci ;
ou dans lequel l'effet thérapeutique est comprise entre 1 et 400 mg du composé ou d'un sel pharmaceutiquement acceptable de celui-ci ;
ou dans lequel la quantité thérapeutiquement efficace est comprise entre 1 et 300 mg du composé ou d'un sel pharmaceutiquement acceptable de celui-ci ;
ou dans lequel la quantité thérapeutiquement efficace est comprise entre 1 et 200 mg du composé ou d'un sel pharmaceutiquement acceptable de celui-ci ;
ou dans lequel la quantité thérapeutiquement efficace est comprise entre 1 et 100 mg du composé ou d'un sel pharmaceutiquement acceptable de celui-ci ;
ou dans lequel la quantité thérapeutiquement efficace est de 10 mg du composé ou d'un sel pharmaceutiquement acceptable de celui-ci ;
ou dans lequel la quantité thérapeutiquement efficace est de 30 mg du composé ou d'un sel pharmaceutiquement acceptable de celui-ci ;
ou dans lequel la quantité thérapeutiquement efficace est de 100 mg du composé ou d'un sel pharmaceutiquement acceptable de celui-ci ;
ou dans lequel la quantité thérapeutiquement efficace est de 200 mg du composé ou d'un sel pharmaceutiquement acceptable de celui-ci ;
ou dans lequel la quantité thérapeutiquement efficace est de 300 mg du composé ou d'un sel pharmaceutiquement acceptable de celui-ci ;
ou dans lequel la quantité thérapeutiquement efficace est de 400 mg du composé ou d'un de ses sels pharmaceutiquement acceptables ;
ou dans lequel la quantité thérapeutiquement efficace est de 600 mg du composé ou d'un sel pharmaceutiquement acceptable de celui-ci ;
ou dans lequel la quantité thérapeutiquement efficace est de 800 mg du composé ou d'un sel pharmaceutiquement acceptable de celui-ci ;
ou dans lequel la quantité thérapeutiquement efficace est de 1000 mg du composé ou d'un sel pharmaceutiquement acceptable de celui-ci ;
ou dans lequel la quantité thérapeutiquement efficace est de 1200 mg du composé ou d'un sel pharmaceutiquement acceptable de celui-ci.

3. Composé destiné à être utilisé selon la revendication 1 ou 2, dans lequel la quantité thérapeutiquement efficace est comprise entre 1 et 1000 mg du composé ou d'un sel pharmaceutiquement acceptable de celui-ci.

4. Composé destiné à être utilisé selon la revendication 1 ou 2, dans lequel la quantité thérapeutiquement efficace est comprise entre 1 et 800 mg du composé ou d'un sel pharmaceutiquement acceptable de celui-ci.

5. Composé destiné à être utilisé selon la revendication 1 ou 2, dans lequel la quantité thérapeutiquement efficace est comprise entre 1 et 600 mg du composé ou d'un sel pharmaceutiquement acceptable de celui-ci.

6. Composé destiné à être utilisé selon la revendication 1 ou 2, dans lequel la quantité thérapeutiquement efficace est comprise entre 1 et 400 mg du composé ou d'un sel pharmaceutiquement acceptable de celui-ci.

7. Composé destiné à être utilisé selon la revendication 1 ou 2, dans lequel la quantité thérapeutiquement efficace est de 200 mg.

8. Composé destiné à être utilisé selon la revendication 1 ou 2, dans lequel la quantité thérapeutiquement efficace est de 300 mg.

9. Composé destiné à être utilisé selon la revendication 1 ou 2, dans lequel la quantité thérapeutiquement efficace est de 600 mg.

10. Composé destiné à être utilisé selon l'une quelconque des revendications 1 à 9, dans lequel le composé ou un sel pharmaceutiquement acceptable de celui-ci est administré une fois par jour.

11. Composé destiné à être utilisé selon l'une quelconque des revendications 1 à 9, dans lequel le composé ou un sel pharmaceutiquement acceptable de celui-ci est administré deux fois par jour.

12. Composé destiné à être utilisé selon l'une quelconque des revendications 1 à 9, dans lequel le composé ou un sel pharmaceutiquement acceptable de celui-ci est administré trois fois par jour.

13. Composition destinée à être utilisée selon l'une quelconque des revendications 1 à 12, dans lequel le composé est 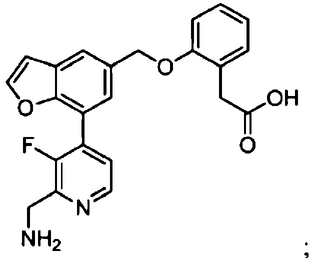 ou un sel pharmaceutiquement acceptable de celui-ci.

14. Composition destinée à être utilisée selon l'une quelconque des revendications 1 à 12, dans lequel le composé est 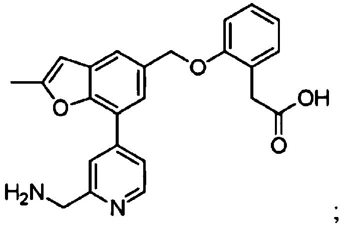 ou un sel pharmaceutiquement acceptable de celui-ci.
